(19) **Europäisches Patentamt**
**European Patent Office**
**Office européen des brevets**

(11) **EP 4 386 767 A1**

(12) # EUROPEAN PATENT APPLICATION

(43) Date of publication:
   **19.06.2024   Bulletin 2024/25**

(21) Application number: **22306927.9**

(22) Date of filing: **16.12.2022**

(51) International Patent Classification (IPC):
   **G16H 10/20** (2018.01)      **G16H 50/20** (2018.01)
   **G16H 50/70** (2018.01)

(52) Cooperative Patent Classification (CPC):
   **G16H 50/20; G16H 10/20; G16H 50/70**

(84) Designated Contracting States:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Designated Extension States:
   **BA**
   Designated Validation States:
   **KH MA MD TN**

(71) Applicant: **Dassault Systèmes**
   **78140 Vélizy-Villacoublay (FR)**

(72) Inventors:
   • **COTTIN, AZILIZ**
     **78946 Vélizy-Villacoublay Cedex (FR)**

   • **PECUCHET, NICOLAS**
     **78946 Vélizy-Villacoublay Cedex (FR)**
   • **ZULIAN, MARINE**
     **06410 Biot (FR)**
   • **KATSAHIAN, Sandrine**
     **75015 Paris (FR)**
   • **GUILLOUX, Agathe**
     **75015 Paris (FR)**

(74) Representative: **Bandpay & Greuter**
   **11 rue Christophe Colomb**
   **75008 Paris (FR)**

(54)   **CHARACTERISTICS OF PATIENT INFLUENCING DISEASE PROGESSION**

(57)   The disclosure notably relates to a computer-implemented method for determining characteristics of a patient that influence a progression of a disease of the patient. The method comprises, while training a neural network with a provided dataset, for each transition of the multi-state model, and for each characteristic, determining S30 a respective quantification of an impact of the characteristic on the results of the neural network. The method comprises, for each transition, identifying S41 a list of characteristics of the set of characteristics, and, for each given characteristic of the identified list, determining S42 a relationship between the given characteristic and probabilities of transition. The method comprises providing 550 the identified lists and the determined relationships that influence the progression of the disease of the patient. Such a method forms an improved solution for determining patient's characteristics that influence patient disease progression.

FIG. 1

**EP 4 386 767 A1**

# EP 4 386 767 A1

**Description**

**TECHNICAL FIELD**

The disclosure relates to the field of biostatistics, and more specifically to methods, system and program for determining characteristics of a patient that influence a progression of a disease of the patient.

**BACKGROUND**

A clinical setting aims to provide a prediction of a probability of occurrence of successive clinical events (for example relapse or death), conditionally to potential risk factors (i.e., "covariates"). These covariates are baseline (i.e., measured at the beginning of a patient's follow-up period). To model patient's histories when multiple events can occur successively over time, the multi-state approach [1] has been developed as a generalization of classical survival models for more complex disease progression. A multi-state model is a continuous-time stochastic process where the events are represented by states and the probability of occurrence of the events by transitions between the states. An example of a multi-state model is, e.g., described in the European patent application EP 4057297A1published on 14.09.2022. As detailed in this European patent application EP 4057297A1, a substantial contribution of the multi-state model is the flexible modeling of the relation between covariates and risks of transitions from one state to another. This leads to finer individual estimation of the probabilities of transiting between the states when nonlinear patterns are found in the data.

Interpreting the survival model is essential to address healthcare and clinical challenges. In particular, a good understanding of patient's characteristics that influence patient disease progression is crucial, e.g., for designing clinical trials or for providing better patient's follow-up periods. When using classical survival methods such as the Cox P.H. model [2], the model, which is not machine learning based, is easily interpretable by analyzing the transition-specific coefficients. However, models that are machine learning based, such as deep learning models, require model-agnostic methods to be interpretable.

To make machine learning interpretable, one way is to use model-specific interpretability approaches. Neural Interpretation Diagram (NID) is a standard method for interpreting classical neural networks. This method allows a visual interpretation of the connection weights among the neurons of the network. Tracking the magnitude and direction of weights between neurons allows identifying effects of the input features on the model outcome. Then, to compute the relative contribution of each feature, the Garson's algorithm [4] may compute a global weight of the connections that the feature has crossed.

In practice for simple models with a single hidden layer and few neurons, number of paths in the graph is short and it is therefore feasible to explicitly compute the contribution of each variable. In the case of deep models (characterized by a high number of hidden layers and a high number of neurons), it is not possible to use this method.

To address this problem, model-agnostic interpretation methods [5] have been developed. These methods can be applied to any deep learning model as they analyze the model after training (i.e., post hoc methods). The idea of these methods is to measure variations in the model outcome when changing the model input.

There are two main literature streams of model-agnostic methods. The first one is based on computing a quantitative importance of each feature, which includes state-of-the-art methods based on Permutation Feature Importance [6] (PFI) and Leave-One-Covariate-Out [7] (LOCO). The second one proposes graphical tools to interpret the nature of relationship between the input features and the outcome ; it includes state-of-the-art methods based on Partial Dependence Plots [8] (PDP) and Accumulated Local Effects [9] (ALE). The PDP provides a graphical interpretation of the average marginal effect of a certain feature on the predicted outcome of the model. The equivalent to PDP to measure individual effects of the features is called Individual Conditional Expectation [10] (ICE) plots. An ICE plot provides a graphical interpretation of relationship between the prediction and a feature for each instance separately.

Other methods such as LIME [11] (Local interpretable Model-Agnostic Explications) explains individual predictions by locally approximating the predictions using a less complex and interpretable model, called a surrogate model, and can be for example a Cox P.H. model. In addition, SHAP [12] (SHapley Additive exPlanations) is a method to explain individual predictions based on the Shapley values computation. This method is often used for tree-based methods.

Main limitations of these methods are that they provide biased interpretation of the results of the model, and that they do not objectively and accurately identify the important characteristics for each transition and their influence on the results provided by the network.

Within this context, there is still a need for an improved solution for determining patient's characteristics that influence patient disease progression.

**CITED REFERENCES**

[1] Per K Andersen, Ornulf Borgan, Richard D Gill, and Niels Keiding. Statistical models based on counting processes. Springer Science & Business Media, 2012.

[2] David R Cox. Regression models and lifetables. Journal of the Royal Statistical Society: Series B (Methodological), 34(2): 187-202, 1972.

[3] Stuart J. Pocock, PHD, Tim C. Clayton, MSC, Gregg W. Stone, MD. 2015. Design of Major Randomized Trials: Part 3 of a 4-Part Series on Statistics for Clinical Trials.

[4] Garson, G.D. 1991. Interpreting neural network connection weights. Artificial Intelligence Expert. 6(4):46-51.

[5] Christoph Molnar. Interpretable machine learning. Lulu. com, 2020.

[6] Breiman, Leo."Random Forests." Machine Learning 45 (1). Springer: 5-32 (2001).

[7] Lei, Jing, Max G'Sell, Alessandro Rinaldo, Ryan J Tibshirani, and Larry Wasserman. 2018. "Distribution-Free Predictive Inference for Regression." Journal of the American Statistical Association 113 (523). Taylor & Francis: 1094-1111.

[8] Friedman, Jerome H. "Greedy function approximation: A gradient boosting machine." Annals of statistics (2001): 1189-1232.

[9] Apley, Daniel W., and Jingyu Zhu. "Visualizing the effects of predictor variables in black box supervised learning models." Journal of the Royal Statistical Society: Series B (Statistical Methodology) 82.4 (2020): 1059-1086.

[10] Goldstein, Alex, Adam Kapelner, Justin Bleich, and Emil Pitkin. "Peeking inside the black box: Visualizing statistical learning with plots of individual conditional expectation." journal of Computational and Graphical Statistics 24, no. 1 (2015): 44-65.

[11] Tulio Ribeiro, M., Singh, S., & Guestrin, C. (2016). "Why Should I Trust You?": Explaining the Predictions of Any Classifier.

[12] Lundberg, Scott M., and Su-In Lee. "A unified approach to interpreting model predictions." Advances in Neural Information Processing Systems. 2017.

[13] C Molnar, S Gruber, and P Kopper. Limitations of interpretable machine learning methods, 2020

[14] John Ehrlinger. ggrandomforests : Exploring random forest survival. arXiv preprint arXiv :1612.08974, 2016.

[15] Maxim S Kovalev, Lev V Utkin, and Ernest M Kasimov. Survlime : A method for explaining machine learning survival models. Knowledge-Based Systems, 203 :106164, 2020.

[16] Richard Li, Ashwin Shinde, An Liu, Scott Glaser, Yung Lyou, Bertram Yuh, Jeffrey Wong, and Arya Amini. Machine learning-based interpretation and visualization of nonlinear interactions in prostate cancer survival. JCO Clinical Cancer Informatics, 4 :637-646, 2020.

[17] Cottin, A., Pecuchet, N., Zulian, M., Guilloux, A., & Katsahian, S. (2022). IDNetwork: A deep illness-death network based on multi-state event history process for disease prognostication. Statistics in Medicine, 41(9), 1573-1598.

[18] Ishwaran H and Kogalur UB. Random survival forests for r. R news 2007; 7(2): 25-31.

[19] Ishwaran H, Gerds TA, Kogalur UB et al. Random survival forests for competing risks. Biostatistics 2014; 15(4): 757-773.

[20] Jacqmin-Gadda H, Blanche P, Chary E, Touraine C, Dartigues J-F. Receiver operating characteristic curve estimation for time to event with semicompeting risks and interval censoring. Stat Methods Med Res. 2016;25(6):2750-2766.

[21] Spitoni C, Lammens V, Putter H. Prediction errors for state occupation and transition probabilities in multi-state models. Biom J. 2018;60(1):34-48.

[22] Pepe MS and Mori M. Kaplan-meier, marginal or conditional probability curves in summarizing competing risks failure time data? Statistics in medicine 1993; 12(8): 737-751.

[23] Ramezankhani, A., Blaha, M.J., Mirbolouk, M.h. et al. Multi-state analysis of hypertension and mortality: application of semi-Markov model in a longitudinal cohort study. BMC Cardiovasc Disord 20, 321 (2020). https://doi.org/10.1186/s12872-020-01599-7.

[24] Milic J, Banchelli F, Meschiari M, Franceschini E, Ciusa G, et al. (2021) The impact of tocilizumab on respiratory support states transition and clinical outcomes in COVID-19 patients. A Markov model multi-state study. PLOS ONE 16(8): e0251378. https://doi.org/10.1371/journal.pone.0251378.

## SUMMARY

It is therefore provided a computer-implemented method for determining characteristics of a patient that influence a progression of a disease of the patient. This method is referred to in the following as "the method". The method comprises providing a deep learning neural network modeling a multi-state model of the disease progression. The multi-state model comprises states and transitions between the states. The multi-state model outputs transition-specific probabilities during a follow-up period. The method comprises providing a dataset of multi-state time-to-event data of a set of patients. The dataset comprises, for each patient of the set, a real value of each characteristic of a set of characteristics.

The method comprises, while training the neural network with the provided dataset, for each transition of the multi-state model, and for each characteristic, determining a respective quantification of an impact of the characteristic on the results of the neural network. The determined respective quantification is a sum, for each patient, of a difference between a conditional probability obtained for a baseline value of the characteristic and a conditional probability obtained for the real value of the characteristic for the patient. The method comprises, for each transition, identifying a list of characteristics of the set of characteristics. Each characteristic of the list has a significant respective quantification for the transition. The method comprises, for each transition, and for each given characteristic of the identified list, determining a relationship between the given characteristic and probabilities of transition based on computed predictions of conditional probabilities for each patient of the set of patients. The relationship is determined by fixing each characteristic of the patient to each value of the characteristic. The method comprises providing the identified lists and the determined relationships that influence the progression of the disease of the patient.

The method may comprise one or more of the following:

- the determining (S30) of the respective quantification comprises, for each transition $ql$ from a state $q$ to a state $l$:

  ○ computing an average reference transition-specific conditional probability based on the formula:

$$CP_{ql}(t|X)^{reference} = \frac{1}{N}\sum_{i=1}^{N} CP_{ql}\left(t|X^{(i)}\right)$$

  wherein $X = (X_1, ..., X_p)$ is the set of characteristics, $i$ is the index of each patient of the set ($1 \leq i \leq N$), $CP_{ql}(t|X^{(i)})$ is the conditional probability for the real values of the set of characteristics $X^{(i)}$ for the patient i and $CP_{ql}(t|X)^{reference}$ is the computed average reference transition-specific conditional probability; and

  ○ For each characteristic $j$ ($1 \leq j \leq P$):

    • computing an average perturbated transition-specific conditional probability based on the formula:

$$CP_{ql}(t)^{baseline,j} = \frac{1}{N}\sum_{i=1}^{N} CP_{ql}\left(t|X_{\setminus j}^{(i)}, x_j^{baseline}\right)$$

$$(1 \leq j \leq P), CP_{ql}\left(t|X_{\setminus j}^{(i)}, x_j^{baseline}\right)$$

    wherein $X_{\setminus j}$ is the set of characteristics without the characteristic $X_j$ is a conditional probability obtained for the patient i based on the baseline value $x_j^{baseline}$ for the characteristic $j$ and the real values of the patient for each other characteristic of the set $X_{\setminus j}^{(i)}$ and $CP_{ql}(t)^{baseline,j}$ is the computed average perturbated transition-specific conditional probability; and

    • computing a prediction-based feature importance based on the formula:

$$I_{ql}^{j} = \int_{0}^{t} \left(CP_{ql}(t)^{baseline,j} - CP_{ql}(t|X)^{reference}\right)dt$$

    wherein $I_{ql}^{j}$ is the computed prediction-based feature importance, thereby determining the respective quantification for the characteristic and the transition;

- the baseline value of the characteristic is the same for each of the patients;
- the set of characteristics comprise one or more discrete characteristics and/or one or more continuous characteristics, the real value of each patient being, for each of the one or more discrete characteristics, one of a respective predetermined set of potential values, the baseline value being:

  ∘ for each discrete characteristic, one of the potential values of the respective predetermined set of the discrete characteristic, and
  ∘ for each continuous characteristic, determined according to a scaling of real values of the patients for the characteristic;

- the determining (S42), for each transition $ql$ from the state $q$ to the state $l$, of the relationship for each characteristic of the list comprising:

  ∘ for each potential value $X_{jk}$ ($k = 1, ..., K_j$) of the characteristic $j$:

    ▪ computing a partial dependence measure between the potential value $X_{jk}$ and a risk of transition as an average of individual predictions based on the formula:

$$PDP_{ql}^t(X_{jk}) = \frac{1}{N}\sum_{i=1}^{N} CP_{ql}\left(t|X_{\setminus j}^{(i)}, X_{jk}\right)$$

wherein $X = (X_1, ..., X_p)$ is the set of characteristics, $X_{\setminus j}$ is the set of characteristics without the characteristic $X_j$ ($1 \le j \le P$), $CP_{ql}\left(t|X_{\setminus j}^{(i)}, X_{jk}\right)$ is a conditional probability obtained for the patient $i$ based on the potential value $X_{jk}$ for the characteristic $j$ and the real values of the patient for each other characteristic of the set $X_{\setminus j}^{(i)}$ and $PDP_{ql}^t(X_{jk})$ is the computed partial dependence measure; and
    ∘ determining a partial dependance plot based on the computed partial dependence measures, the partial dependance plot being defined, for each characteristic $j$, as $\left\{X_{jk}, PDP_{ql}^t(X_{jk})\right\}_{t,k}$;

- the partial dependance plot comprises, for each transition, a respective graphical representation, the respective graphical representation representing, for each characteristic of the identified list of the transition, a respective curve representing the risk of transition as a function of time for the characteristic;
- the multi-state model is an illness-death model;
- the method further comprises:

  ∘ while training the neural network with the provided dataset, for each transition of the multi-state model:

    ▪ for each characteristic, determining a respective performance quantification of an impact of the characteristic on performances of the neural network;

  ∘ for each transition:

    ∘ identifying another list of characteristics of the set of characteristics, each characteristic of the another list having a significant respective performance quantification for the transition;
    ∘ selecting, by user interaction, an analysis type among a result type and a performance type, the result type being associated to the list and the performance type being associated to the another list; and
    ∘ providing to the user the lists associated to the analysis type selected by the user; and/or

- a respective quantification of a characteristic is significant when the respective quantification is positive for at least 50% of the patients, preferably 95% of the patients.

It is also provided a method of use of characteristics of the patient that influence the progression of the disease of the patient determined by the method. This method is referred to in the following as "the method of use". The method of use comprises performing subgroups stratification of patients based on the provided lists and relationships that influence the progression of the disease of the patient.

The method of use may further comprise classifying each transition in a first group of transitions or a second group of transitions. The transitions of the first group may be associated with a favorable clinical evolution and the transitions of the second group may be associated with a negative clinical evolution. The method of use may further comprise determining inclusion and exclusion criteria for a survival analysis based on the classified transitions.

The method of use may further comprise determining a follow-up period for one or more patients. The determining of the follow-up period may comprise, for each patient, determining the subgroup of the patient, and, determining a follow-up period for the patient according to the determined subgroup of the patient.

It is further provided a computer program comprising instructions which, when the program is executed by a computer, cause the computer to carry out the method and/or the method of use.

It is further provided a computer readable storage medium having recorded thereon the computer program.

It is further provided a system comprising a processor coupled to a memory. The memory has recorded thereon the computer program.

It is further provided a device comprising a data storage medium having recorded thereon the computer program.

The device may form or serve as a non-transitory computer-readable medium, for example on a SaaS (Software as a service) or other server, or a cloud based platform, or the like. The device may alternatively comprise a processor coupled to the data storage medium. The processor may execute the instructions of the computer program stored on the data storage medium to cause the device to carry out the method and/or the method of use. The device may thus form a computer system in whole or in part (*e.g.* the device is a subsystem of the overall system). The system may further comprise a graphical user interface coupled to the processor.

## BRIEF DESCRIPTION OF THE DRAWINGS

Non-limiting examples will now be described in reference to the accompanying drawings, where:

- FIG. 1 shows a flowchart of an example of the method;
- FIG. 2 illustrates the fundamentals of design for major randomized controlled trials;
- FIG.s 3 to 5 illustrate examples of implementations of the method;
- FIG. 6 illustrate example of visualization of the method;
- FIG.s 7 and 8 show examples of the graphical representation of the partial dependance plot;
- FIG.s 9 to 11 illustrate examples of the method of use; and
- FIG. 12 shows an example of the system.

## DETAILED DESCRIPTION

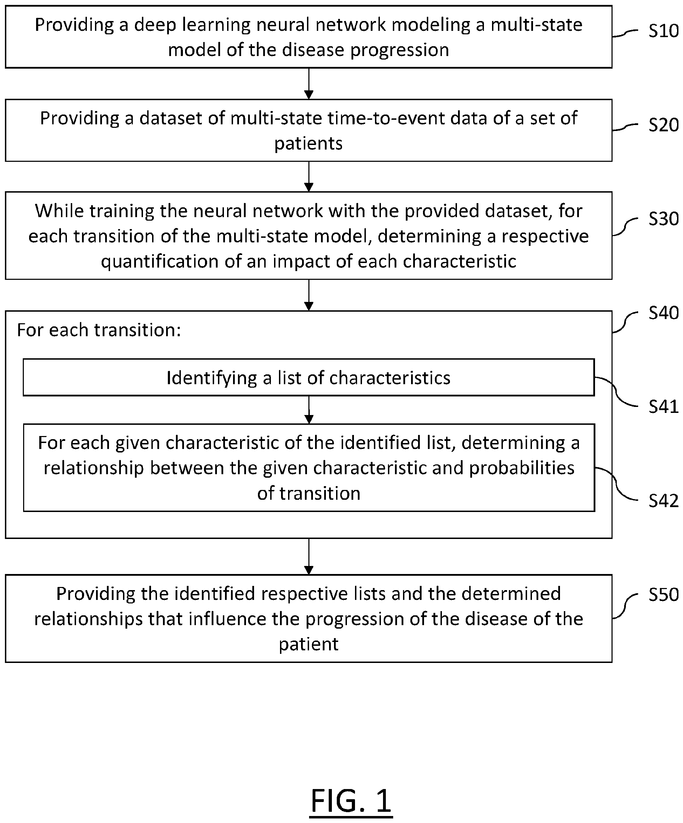

With reference to the flowchart of FIG. 1, it is provided a computer-implemented method for determining characteristics of a patient that influence a progression of a disease of the patient. The method comprises providing S10 a deep learning neural network modeling a multi-state model of the disease progression. The multi-state model comprises states and transitions between the states. The multi-state model outputs transition-specific probabilities during a follow-up period. The method comprises providing S20 a dataset of multi-state time-to-event data of a set of patients. The dataset comprises, for each patient of the set, a real value of each characteristic of a set of characteristics. The method comprises, while training the neural network with the provided dataset, for each transition of the multi-state model, and for each characteristic, determining S30 a respective quantification of an impact of the characteristic on the results of the neural network. The determined respective quantification is a sum, for each patient, of a difference between a conditional probability obtained for a baseline value of the characteristic and a conditional probability obtained for the real value of the characteristic for the patient. The method comprises, for each transition, identifying S41 a list of characteristics of the set of characteristics. Each characteristic of the list has a significant respective quantification for the transition. The method comprises, for each transition, and for each given characteristic of the identified list, determining S42 a relationship between the given characteristic and probabilities of transition based on computed predictions of conditional probabilities for each patient of the set of patients. The relationship is determined by fixing each characteristic of the patient to each value of the characteristic. The method comprises providing S50 the identified lists and the determined relationships that influence the progression of the disease of the patient.

Such a method forms an improved solution for determining patient's characteristics that influence patient disease progression.

Notably, the method allows interpreting the results predicted by the deep learning neural network modeling the multi-state model. The method is adapted for the interpretation of the multi-state model that the neural network models: indeed, the method provides, for each transition of the multi-state model, the list of characteristics that have significant impact(s) on the results of the neural network for the transition. The method therefore allows determining which are the patient's characteristics that influence the results of the neural network for each transition. Providing an interpretation of the learning neural network is essential in clinical applications where interpretability and reliability are evaluated to support medical decisions. The method allows this interpretability in order to help clinicians in establishing patient prognosis. The method reveals what are the significant characteristics of patients that are associated with each transition and increases the understanding of the evolution of the disease.

Moreover, the interpretation provided by the method is objective. Indeed, the interpretation provided by the method is based on respective quantifications that are determined for each characteristic and that objectively assess the impact of each characteristic on the results of the neural network. In particular, the respective quantification is a sum, for each patient, of a difference between a conditional probability obtained for a baseline value of the characteristic and a conditional probability obtained for the real value of the characteristic for the patient. Each respective quantification thus allows quantifying an impact on the obtained conditional probability (i.e., on the results of the neural network) of the value of the characteristic, from a difference between when the real value is used and when a baseline value is used. The interpretation provided by the method is therefore particularly robust and reliable as it is based on objective quantifications.

Furthermore, for each characteristic of the list identified for each transition, the method determines a relationship between the characteristic and the probabilities of transition. The method is therefore able to assess, in addition to the lists of characteristics, the influence of each characteristic on the probabilities of transition. The method therefore outputs a complete and exhaustive interpretation of the deep learning neural network, especially of the influence of each characteristic that significantly impacts the results of the neural network.

Additionally, the computing of the lists and of the relationships is carried out automatically by a computer system. This allows for a deterministic and accurate computation of the lists and of the relationships that influence the progression of the disease of the patient. In particular, this allows a user, e.g., a doctor, to obtain the lists and the relationships without tedious efforts: the user provides the deep learning neural network and the dataset and the computer automatically provides the identified lists and the determined relationships that influence the progression of the disease of the patient. This makes the method ergonomic.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory the memory having recorded thereon a computer program comprising instructions for performing the method. The memory may also store the database. The memory is any hardware adapted for such storage, possibly comprising several physical distinct parts (e.g. one for the program, and possibly one for the database). The system may comprise a graphical user interface (GUI) for displaying to a user representations of data computed and/or provided and/or obtained in the course of the method.

It is also provided a method of use of characteristics of the patient that influence the progression of the disease of the patient determined by the above-discussed method. The above-discussed method may be included in the method of use. For example, the method of use may comprise performing the method, and, then, may comprise the performing of the subgroups stratification of patients based on the provided lists and relationships that influence the progression of the disease of the patient. Alternatively, the method may not be included in the method of use. For example, the method may have been performed prior to the method of use. In that case, the results of the method may have been recorded (i.e., the provided lists and relationships that influence the progression of the disease of the patient), e.g., on the memory, and the method of use may comprise, prior to the performing of the subgroups stratification of patients, retrieving the results of the method recorded (e.g., on the memory). Then, the method of use may comprise performing the subgroups stratification of patients based the retrieved results (i.e., on the lists and the relationships retrieved from the memory).

The performing of the subgroups stratification of patients may comprise creating the subgroups of patients of the stratification. The creating of the subgroups of patients may be based on transition-specific probabilities of each patient. Each created subgroup may gather patients having similar transition-specific probabilities (e.g., transition-specific probabilities that are close, i.e., included in a same range of probabilities). The performing of the subgroups stratification may be based on a time series clustering (e.g., time series K-means clustering). Then, the method of use may comprise analyzing the characteristics of the patients of each created subgroup, e.g., with a factor analysis for mixed data (FAMD analysis). The analyzing may comprise exploring characteristics of patients of each subgroup (e.g., each risk cluster) and determining potential correlations between characteristics.

The method of use may further comprise interpreting each transition of the multi-state model. Transitions may be classified in two groups: a first group of favorable transitions and a second group of unfavorable transitions. Favorable transitions of the first group are associated with a favorable clinical evolution. For example, the favorable clinical evolution may be a complete response to a treatment. The transitions of the second group may be associated with a negative clinical evolution. For example, the negative clinical evolution may be a relapse.

Then, the method of use may comprise determining inclusion and exclusion criteria for a survival analysis based on the classified transitions. For example, the method of use may comprise determining inclusion criteria based on the list of characteristics of each transition in the first group (i.e., associated with a favorable clinical evolution). The method of use may comprise using the determined inclusion criteria for patient selection in a clinical trial or in a care procedure (e.g., treatment, intervention and follow-up period). Alternatively or additionally, the method of use may comprise determining exclusion criteria based on the list of characteristics of each transition in the second group (i.e., associated with a negative clinical evolution). The method of use may comprise using the determined exclusion criteria for patient exclusion from a clinical trial or from a care procedure (e.g., treatment, intervention and follow-up period). For example, the inclusion and exclusion criteria may be used to assist (e.g., a medical practitioner such as a physician or doctor) in selecting or excluding patients for a clinical trial.

The method of use may further comprise designing a clinical study according to the determined inclusion and exclusion criteria. For example, the designing may comprise deciding which patients to include in the clinical study based on the determined inclusion and exclusion criteria (i.e., including patients who meet the inclusion criteria and excluding patients who meet the exclusion criteria). The method of use may further comprise simulating the designed clinical study, and, optionally, checking that the simulated clinical study is an unbiased study. The checking may comprise verifying propensity scores indicating a balance of patient's characteristics between arms of the clinical study. This allows ensuring that patients in control and experimental arms of the simulated clinical study have balanced characteristics across arms.

The method of use may further comprise determining a follow-up period for one or more patients. The determining of the follow-up period may be performed after the performing of the subgroups stratification and may be based on the determined subgroups of the patient. The determining of the follow-up period may comprise, for each patient, determining the subgroup of the patient, and, determining a follow-up period for the patient according to the determined subgroup of the patient. The determining of the subgroup of the patient may be based on the values of the characteristics of the patient. For example, each subgroup may be associated with values and/or ranges of values (e.g., for continuous characteristics) for one or more of the characteristics, and the determining may comprise evaluating (e.g., successively for each subgroup) whether the values of the characteristics of the patient are equal to the values and/or ranges of values associated with the subgroup. If the evaluating is positive for a given subgroup, the determining may identify the given subgroup as the subgroup of the patient.

Then, the method of use may comprise determining a follow-up period for the patient according to the determined subgroup of the patient of use. For example, each subgroup of patient may be associated with a follow-up period (e.g., that has been evaluated based on results of clinical studies prior), and the follow-up period determined may be the follow-up period associated with the subgroup of the patient. The follow-up period associated with each subgroup may be recorded, e.g., on a memory, and the method of use may comprise retrieving from the memory the follow-up period associated with the subgroup of the patient. The determined follow-up period may be a recommendation to a medical practitioner such as a physician or doctor. The determined follow-up period may be used to assist (e.g., the medical practitioner such as the physician or the doctor) in selecting a treatment to be administered or medical surveillance to be carried out for the patient.

Referring back to the flowchart of FIG. 1, further explanations of the computer-implemented method for determining characteristics of a patient that influence a progression of a disease of the patient are now provided.

The providing S10 of the deep learning neural (e.g., artificial) network modeling the multi-state model is now discussed in more details.

The multi-state model models the progression of a disease. For example, the disease may be a cancer disease, for example a breast cancer, or a disease characterized by an intermediate progression state(s) and a final state. The multi-state model may be the multi-state model described in the European patent application EP 4057297A1 published on 14.09.2022, which is incorporated herein by reference. In particular, the multi-state model may be the multi-state model described from p.9, li.11 to p.10, li.8 of the description as filed of this European patent application EP 4057297A1. In examples, the multi-state model may be an illness-death model. In that case, the multi-state model may comprise three states ("healthy", "relapsed" or "diseased" and "death") and three transitions between these states (i.e., a first transition between the state "healthy" and the state "relapsed" or "diseased", a second transition between state "relapsed" or "diseased" and the state "death" and a third transition between the state "healthy" and the state "death"). For example, the multi-state model may be the illness-death model described in the European patent application EP 4057297A1 (referred to as the "IDNetwork" model in the following), which is incorporated herein by reference. In particular, the multi-state model may be the illness-death model described in p.10 from li.9 to 32 or from p.16, li.9 to p.18, li.11 of the description as filed of this European patent application EP 4057297A1. In other examples, the multi-state model may comprise more than three states. In that case, the multi-state model may comprise a transition between each pair of states. Each transition is specific between two respective states (i.e., the two respective states of the pair).

The output of the multi-state model are transition-specific probabilities during a follow-up period. For example, the multi-state model may take as input real values of characteristics of a patient, and may output the transition-specific probabilities during a follow-up period for the patient. The outputted transition-specific probabilities may be function of time. For example, the output may be a distribution of transition-specific probabilities for each interval of a set of intervals. The set of intervals may be such that it forms a subdivision of the follow-up period. In other words, the output is a distribution of probabilities constant values, with exactly one probability constant value per each respective time interval of a follow-up period and per each respective transition of the multi-state model. Said probability constant value (e.g., a single number, e.g., between 0 and 1) represents probability that the patient experiences the respective transition (and thus corresponding changes states) during said respective time interval.

The providing S20 of the dataset is now discussed in more details.

The dataset may comprise multi-state time-to-event data of a set of patients. For example, the set of patients may comprise more than 10 patients, e.g., more than 100 or 1000 patients. Each of the patient may be a real patient, and the time-to-event data may be time-to-event data that the real patient has experienced. The time-to-event data may include one or more times each corresponding to a transition between two states of the multi-state model for the patient (e.g., the time of transition between diseased or death).

For each patient of the set, the dataset comprises a real value of each characteristic of a set of characteristics. For example, the set of characteristics may comprise more than 3 characteristics, e.g., more than 10 or 25 characteristics. Each characteristic may be a medical characteristic, i.e., may relate to the physiology of the patient. The set of medical characteristics may comprise characteristics representing a general state of the patient and/or characteristics representing a condition of the patient with respect to the illness. The set of medical characteristics may also comprise characteristics representing a general state of the patient disease (e.g., for cancer patient this may be the tumor size or the cancer stage). The set of characteristics may comprise one or more discrete characteristics (e.g., a gender, an ethnicity and/or a general health status). The real value of each patient may be, for each discrete characteristic, one of a respective predetermined set of potential values. Alternatively or additionally, the set of characteristics may comprise one or more continuous characteristics (e.g., an age, a height and/or a weight). For each characteristic, the dataset may comprise the real value of each characteristic for the real patient. The real value of each patient may be, for each continuous characteristic, included inside a respective predetermined interval of potential values.

The providing S20 of the dataset may comprise retrieving the dataset from a memory (e.g., on which the dataset is stored). The dataset may have been formed and/or recorded on the memory prior to the executing of the method. For example, the real values of the set of characteristics may have been determined for each patient prior to the executing of the method. The real values may have been determined and/or measured on the patients (e.g., by a medical practitioner such as a physician or doctor) and then may have been recorded on the memory prior to the executing of the method.

The determining S30 of the respective quantifications during the training of the neural network is now discussed in more details.

The method may comprise the training of the neural network. In that case, the method may perform the determining of the quantifications at the same time than the performing of the training of the neural network. Alternatively, the training of the neural network may be performed in parallel to the performing of the method. For example, the method is executed in parallel to another process that comprises the training of the neural network. In that case, the method may perform the determining of the quantifications at the same time than the training of the neural network is performed by the another process.

The neural network may be the function described in the European patent application EP 4057297A1 published on 14.09.2022, which is incorporated herein by reference. In particular, the neural network may have the architecture of the function that is described from p.12, li.27 to p.14, li.29 of this European patent application EP 4057297A1. The neural network may comprise a covariate-shared subnetwork and/or a transition-specific subnetwork per transition. The covariate-shared subnetwork may comprise a respective fully connected neural network. Alternatively or additionally, at least one transition-specific subnetwork may comprise a fully connected neural network. The covariate-shared subnetwork may comprise a respective non-linear activation function. Alternatively or additionally, at least one transition-specific subnetwork comprises a respective non-linear activation function. Each transition-specific subnetwork may followed by a softmax layer. The multi-state model may comprise competing transitions, and the transition-specific subnetworks of the competing transitions may share a common softmax layer.

The training of the neural network may comprises minimizing a loss function. The loss function may include a likelihood term and/or a regularization term. The regularization term may penalize, in weight matrices, first order differences of weights associated with two adjacent time intervals. Alternatively or additionally, the regularization term may penalize, in bias vectors, first order differences of biases associated with two adjacent time intervals.

The determining of the quantifications is performed while the training the neural network is performed. This means that the determination and training steps may each include respective intermediate steps, and that the timing of execution of one or more intermediate steps of the determination may depend on the execution of one or more intermediate steps of the training. For example, each of the determining and the training steps may comprise intermediate steps associated with each characteristic, and, for each characteristic, the execution of the intermediate steps of the determination that are associated with the characteristic may be after the execution of the intermediate steps of the training that are associated with the characteristic. For example, the determining of each quantification may comprise computing a sum of subtractions of score values without perturbation (i.e., reference) and score values with perturbation for each characteristic, and the training step may comprise computing the score values without perturbation. In that case, after each score value without perturbation computed in the training step, the method may comprise computing the score values with perturbation and performing the computing of the sum of the subtractions of the score values without and with perturbation just computed.

Each quantification quantifies an impact of the characteristics on the results of the neural network. Each quantification quantifies, on average for all the patients, how many the value of the characteristic modifies the results provided by the neural network. The quantification is determined with respect to a baseline value for the characteristic. The determining of each respective quantification may be based on a Permutation Feature Importance (PFI) algorithm. The quantification quantifies, on average for all the patients, the difference on the result when the baseline value is used and when the real value is used. In particular, the quantification is a sum, for each patient, of a difference between a conditional probability obtained for the baseline value of the characteristic and a conditional probability obtained for the real value of the characteristic for the patient (the conditional probability corresponding to the result provided by the neural network for each value). The conditional probability is the probability that the transition occurs for the patient given a current state of the patient and given the values of the characteristics of the patient. The probability is conditioned by the current state of the patient and the values of the characteristics of the patient (i.e., takes into account them). An example of computing of conditional probability is discussed in [22].

The baseline value of the characteristic may be the same for each of the patients. This allows reducing the disruption that would be induced if a random value were used. For example, for each characteristic, the method may comprise selecting a base line value for the characteristic. Then, during the determining of the respective quantification for the characteristic, the method may comprise using the selected base line value for each patient for the computing of the conditional probabilities of the patients. For each discrete characteristic, the baseline value may be one of the potential values of the respective predetermined set of the discrete characteristic. For example, the method may comprise selecting the base line value randomly among all the potential values of the discrete characteristic, or, alternatively selecting a baseline value that is an average of the potential values. For each continuous characteristic, the method may comprise determining a base line value for the continuous characteristic. For example, the baseline value may be determined according to a scaling of real values of the patients for the characteristic (e.g., may be zero when the values of the characteristic are Z-score normalized, or may be equal to a range of the values divided by 2 when the values of the characteristic are min-max normalized). The advantage is to reduce variance in the computation of the quantifications as the way each characteristic is perturbed does not contains randomness and ensures reproducibility and stability in the determining of the quantifications of the characteristics.

The identifying S41 of the lists and the determining S42 of the relationships are now discussed in more details.

The identifying S41 of the lists and the determining S42 of the relationships may be performed after the training of the neural network. For each transition, the identifying of the lists may comprise, for each characteristic, determining whether the respective quantification of the characteristic is significant for the transition. A respective quantification of a characteristic may be significant when the respective quantification is positive for a majority of the patients. The majority of the patients is reached for at least more than 50% of the patients; preferably, the majority of the patients is reached when 90% of the patients are concerned, more preferably the majority of the patients is reached when 95% of the patients are concerned. For example, the determining of whether the respective quantification is significant may comprise determining a percentage of the patients that have a positive impact on the respective quantification (i.e., that increase the respective quantification), and the respective quantification may be significant when this percentage is higher that a threshold percentage (e.g., a predetermined value such as 50% or 95%). Alternatively or additionally, the determining of whether the respective quantification is significant may be based on a statistical test. Alternatively or additionally, the determining of whether the respective quantification is significant may be based on the respective quantification itself. For example, the evaluating may comprise comparing the respective quantification with a threshold value, and determining that the respective quantification of the characteristic is significant when the respective quantification is higher than the threshold value.

The determining S42 of the relationships may be based on a Partial Dependence Plot (PDP) algorithm. PDP algorithms are discussed hereinbelow. For each characteristic of the identified list, the determining S42 of the relationship may comprise computing the predictions of the conditional probabilities of each patient of the set of patients. For each patient, the computed predictions may comprise, for each given characteristic, and each potential value of the given characteristic, a prediction of the transition probability for the potential value of the given characteristic and for the real values of the patient of the other characteristics. For each patient, the computed predictions thus comprise, for each potential value, a prediction for which the given characteristic is fixed to the potential value. Each relationship is thus determined by fixing each characteristic of the patient to each value of the characteristic. Then, the determining S42 of the relationship may comprise averaging the predictions over the patients, thereby obtaining, for each given characteristic, and each potential value of the given characteristic, an average transition probability for the patients.

The providing S50 of the identified respective lists and the determined relationships is now discussed in more details.

The providing S50 may comprise recording the identified respective lists and the determined relationships, e.g., on the memory. The identified respective lists may be recorded as a table, e.g., gathering the characteristics of each respective list determined for each transition. The determined relationships may also be recorded as a table gathering the computed predictions of conditional probabilities for each patient of the set of patients. The providing S60 may also comprise displaying the identified respective lists and/or the determined relationships, e.g., on the graphical user interface. For example, the identified respective lists may be associated with a first display mode and the determined relationships may be associated with a second display mode, and the providing S60 may comprise selecting a display mode among the first display mode and the second display mode and displaying the identified respective lists when the first display mode is selected and the determined relationships when the second display mode is selected. The providing S60 may comprise an alternative display of these two display modes during successive interactions of the user on the two display modes. The providing S60 may also comprise displaying both display modes at the same time, for example when both display modes are selected. The selection of the display mode may be performed by means of icons that are displayed on the graphical user interface and that each represent a respective mode.

In the first display mode, the displaying of the respective identified respective lists may comprise displaying, for each transition, the respective list of characteristics identified for the transition, for example near a displayed icon representing the transition, or upon user interaction on the icon representing the transition. The displaying of the respective identified respective lists may further comprise, for each displayed list, displaying the respective quantification of each characteristic of the displayed list (e.g., in the form of a score associated with the characteristic). In the second display mode, the displaying of the determined relationships may comprise, for each transition, displaying a graphical representation of the relationships determined for the transition, e.g., upon user interaction on the displayed icon representing the transition.

With reference to FIG.s 2 to 11, examples of implementations of the method and of the method of use are now discussed.

The method provides an interpretation of patients outcome survival prediction, e.g., for personalized medicine. The method provides interpretability in a medical setting, i.e., derives concise and relevant medical concepts from data. The medical settings may be linked with healthcare and medical practice considering for example medical surveillance or with the clinical trial field where model interpretability can help designing new clinical trials.

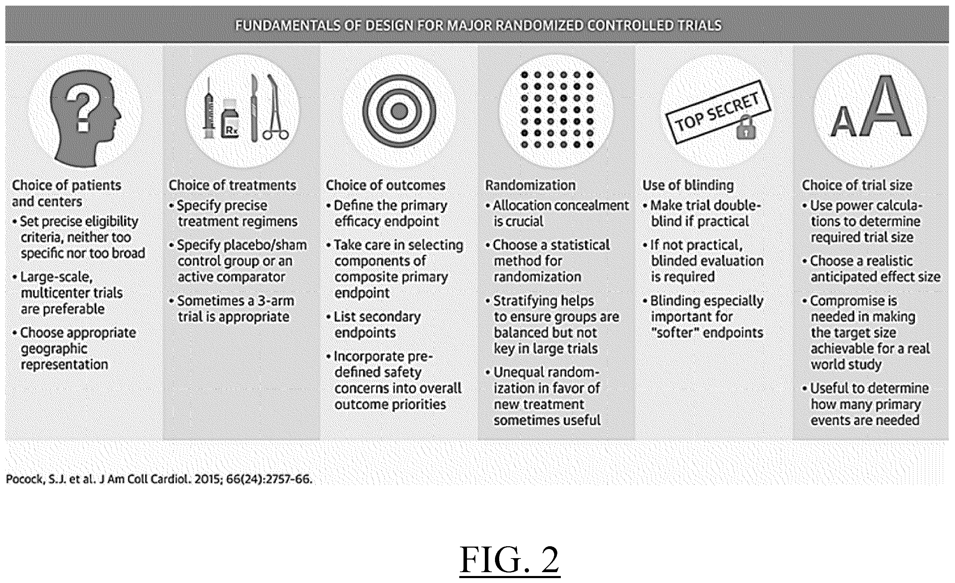

One example of application of the method is the provision of a tool to facilitate the design of clinical trials. For example, the design may comprise deciding which patients to include in the clinical study based on the determined subgroups stratification and/or the determined inclusion and exclusion criteria. The method allows properly designing clinical trials, which provides a highest level of evidence to support new drug or medical device approval (as explained in reference [3]). In particular, the method of use may be applied to the design of phase III trials, which are also called pivotal trials since they are intended to directly influence clinical practice. FIG. 2 illustrates the fundamentals of design for major randomized controlled trials. Phase III trials are performed in a context of higher scale in term of patients and consequently with higher stakes and costs. Phase III trials are essential and need to be designed carefully as it will be very difficult to modify the protocol after launching. That means that researchers need to focus on the essentials of the trial protocol by: (i) defining exactly which patients, (ii) which treatment comparison(s), and (iii) which primary (and secondary) endpoints should be studied. Statistically, researchers must ensure that the trial is large enough so that it is adequately powered to detect (or refute) any treatment differences of clinical importance.

Another example of application of the method in healthcare is to provide a better patient's follow-up. An efficient personalized follow-up requires early detection of a patient's relapse and a good understanding of patient's characteristics that may influence patient monitoring.

In each application, the method allows interpreting the results predicted by the deep learning neural network modeling the multi-state model. Explaining predictions in deep neural networks is challenging but essential in clinical applications where interpretability and reliability are evaluated to support medical decisions. The method adds a model interpretability functionality to the neural network (e.g., the IDNetwork model) in order to help clinicians in establishing the patient prognosis. The method reveals what are the patient characteristics associated with each transition and increase the understanding of the evolution of the disease.

State-of-the-art model-agnostic methods are mainly used in the domain of image classification. Some have been adapted for survival analysis. The PFI and PDP algorithms and the ICE plots have been implemented to interpret random survival forests [14]. SurvLIME [15] extended the method LIME for survival analysis. The SHAP method has been applied to interpret survival prediction in patients with prostate cancer [16].

However, none of these methods has been explicitly adapted for multi-state analysis. In addition, most of these methods are applied separately. Hence, the method proposes a complete framework for interpreting the multi-state model (e.g., the IDNetwork model) in clinical applications in order to understand the relationship between covariates and risks of transitions.

In particular, the method uses several successive interpretability algorithms to analyze multi-state models and thus allow the identification of characteristics (referred to also as features in the following) that influence patient survival. The method of use allows the stratification into patient subgroups and the determination of a list of essential characteristics to answer a medical concern related to the survival of diseased patients.

Advantages of the method are now discussed.

A substantial contribution of the method is the extension of the use of interpretability methods for multi-state models. The pipeline aims at providing medical guidelines to better design clinical trials and make better decision about patient's follow-up period. Moreover, the method identifies and provides lists of characteristics to address medical problems. The interpretability pipeline provides significant improvements in the context of clinical trial design. Results shown treatment efficacy in a phase III study including patients with cancer using the method to stratify patients at the clinical trial inclusion. The method may also be used in other area such as medical surveillance.

In a context of medical decision and clinical trial design support, the method proposes a pipeline for the determination of guidelines for the understanding of complex biological phenomena using existing algorithms. The method is based on Machine Learning (more precisely Deep Learning) algorithms for modeling individual clinical events and predicting individual disease progression based on individual patient characteristics. More precisely, the method provides a deep learning (e.g., artificial) neural network to model a multi-state model (also referred to as "process" in the following) defined by specific transition between states [17]. Input of the network is individual patient's characteristics. Output of the network is an individual prediction of the cumulative incidence function (e.g., CIF) for each specific transition of the model. The CIF is a quantity of interest describing probability of disease progression over time through specific disease states.

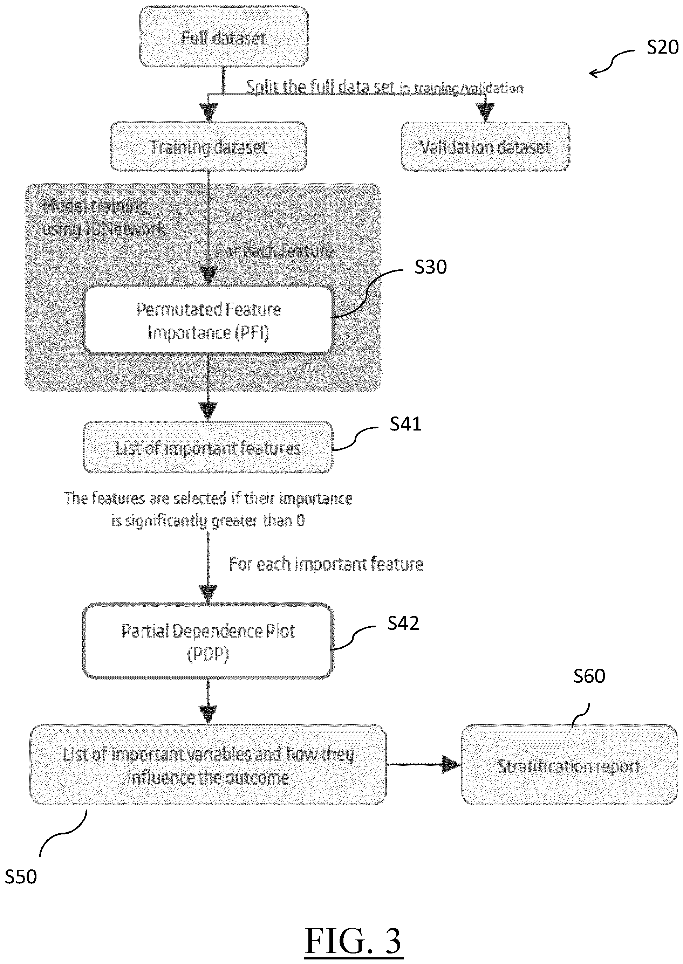

For deep learning methods, often described as black boxes, it is difficult to understand the link between the characteristics considered and the predictions obtained. The method uses cascaded learning interpretation algorithms (illustrated in FIG. 3) for the identification of characteristics (features/variables) that influence disease progression. In particular, the method is based on a one algorithm which is the "Permutation Feature Importance" (PFI) algorithm. The PFI algorithm allows selecting (e.g., automatically) characteristics that are important in the learning. The PFI algorithm selects the characteristics if their importance is significantly greater than 0. Then for these selected characteristics, the method applies the "Partial Dependence Plot" (PDP) algorithm that allow learning the relationship between the outcome and the input characteristics. This means that the PDP algorithm determines the way continuous characteristics (or features), or categories influence the outcome (i.e., the predictions of the multi-state model). The idea of the PDP algorithm is to draw the marginal effect of each input feature (for each of the values that can be taken by the feature) on the predictions. Algorithmically, for one input feature, the PDP algorithm comprises marginalizing the model predictions over the distribution of this feature (i.e., over the space of possible values that can be taken by this feature). It means that, for one input feature and the others input features fixed, the PDP algorithm computing the predictions as in a counterfactual scenario. For example, for a categorical input feature with three modalities, the PDP algorithm comprises displaying an average prediction given each modality of the feature.

In classical multi-state methods such as Cox P.H. models [2] (i.e., in statistical multi-state methods which are not modeled by a deep learning neural network), the use of post-hoc interpretation algorithms is not needed as statistical model coefficients are easily interpretable. Regarding prior-art interpreting methods of machine learning model in the literature, e.g. machine learning model for survival analysis such as random survival forests ([14] and [18]) and random survival forests with competing risks [19], none of them allows interpreting a machine learning model modeling a multi-state model. Additionally, no application of the

The method faces state-of-the-art limitations and allows interpreting the results predicted by the deep learning neural network modeling the multi-state model, as explained in the following.

For each input feature, the respective quantification may be calculated using a perturbation method by randomly permuting the feature values. In that case, the perturbation may depend on shuffling the feature, which adds randomness to the feature importance measure. This can involve a large variance when repeating the permutation. An alternative implementation to reduce variance (referred to as "baseline perturbation") comprises perturbing a feature by replacing its values by a unique value (e.g., as its mean, zero, or an arbitrary value for example). For continuous features, the method may comprise replacing the values according to the feature scaling (e.g., by zero for Z-score normalized features or by range/2 for min-max normalized features). For one-hot encoded features, the method may comprise replacing the values of the feature by zero (i.e., by the reference). The advantage of this alternative implementation is to reduce variance in the computation of the PFI as the way each feature is perturbed does not contains randomness and ensures reproducibility and stability in the estimation of the feature importance measure.

Important features (i.e., having significant respective quantifications) may be different between the transitions of an illness-death model as the evolution of an illness-death process through the states "relapse" and "death" may be due to different clinical factors. In this particular case, transition-specific feature importance is preferred to interpret disease progression. The method therefore advantageously implements transition-specific feature importance and calculates them for each transition of the multi-state model.

Regarding the PFI algorithm, the feature importance is linked to the model error. That means feature importance quantifies how much a feature contribute to the model performance. The method may comprise quantifying the model error in the multi-state process by using discrimination measure with a transition-specific iAUC [20] and using a calibration measure with a transition-specific Brier score [21]. However, in the case of clinical interpretation of a model, it may be interesting to explain how much predictions made by the model vary when the original data is perturbed, rather than considering the impact on the model performance. For this purpose, the method implements a feature importance based on the model predictions. This prediction-based feature importance measure allows to quantify, for each feature, the average effect over time on the risk of a transition. A positive (negative) prediction-based feature importance will indicate an increase (a decrease) of the risk of transition when the feature is perturbed. Hence, the sense of the prediction-based feature importance is interpretable. Whereas by using a model error-based feature importance, a positive feature importance will indicate that the model is sensitive to the perturbation of the feature and therefore this feature plays an important role in the model learning. However, a negative error-based feature importance is not interpretable. We define the prediction-based feature importance as a measure of explicability of the model, whereas the error-based feature importance is a measure of performance of predictability of the model.

Alternatively, both the results of the prediction-based feature importance and the error-based feature importance may be interesting. For this purpose, the method may comprise adding the type of PFI (prediction-based versus error-based) as an option parameter for the user. In that case, the method may further comprise, while training the neural network with the provided dataset, for each transition of the multi-state model, and for each characteristic, determining a respective performance quantification of an impact of the characteristic on performances of the neural network. As for the determining of the quantifications, the determining of the performance quantifications is performed while the training the neural network is performed. Each performance quantification quantifies an impact of the characteristics on the performances of the neural network. Each quantification quantifies, on average for all the patients, how many the value of the characteristic modifies the results provided by the neural network. The performance quantification quantifies (e.g., on average for all the patients) the difference on the performance (or error) when the characteristic is perturbated (i.e., the loss in accuracy and relevance of the predictions provided by the neural network). The determining of each performance quantification may use a discrimination measure with a transition-specific iAUC (as detailed, e.g., in [20]) and use a calibration measure with a transition-specific Brier score (as detailed, e.g., in [21]).

Then, for each, the method may comprise identifying another list of characteristics of the set of characteristics, each characteristic of the another list having a significant respective performance quantification for the transition. The identifying of the another lists may be similar to the identifying of the lists but using the performance quantification instead of the quantification.

After the identifying of the another lists, the method may comprise selecting, by user interaction, an analysis type among a result type and a performance type. For example, the method may comprise selecting, by user interaction, the performance type. The result type is associated to the list and the performance type is associated to the another list. For example, the method may comprise display an icon (e.g., on the graphical user interface) associated with each result type, and the selecting may be performed on the displayed icons. Alternatively, the selection may be performed using a keyboard shortcut for example. After the selection, the method may comprise providing to the user the lists associated to the analysis type selected by the user. For example, the method may comprise displaying the list associated to the analysis type selected by the user (e.g., on the graphical user interface). For example, after the selecting of the performance type, the method may comprise providing to the user the identified another list of each transition.

After the providing of the another lists, the method may comprise, for each identified another list, and for each given characteristic of the identified another list, determining a relationship between the given characteristic and probabilities of transition based on computed predictions of conditional probabilities for each patient of the set of patients. The relationship may be determined by fixing each characteristic of the patient to each value of the characteristic. The determining of the relationship for the characteristics of the another list may be similar to the determining of the relationship for the characteristics of the list (step S42). After the determining of the relations, the method may comprise providing the identified another lists and the determined relationships for the characteristics of these identified another lists (the providing may be similar to the providing S50 of the list discussed above).

In multi-state model, predictions of interest may be the cumulative incidence functions (CIFs). For some transitions, these functions may be conditioned by other transitions (i.e., competing transitions). Thus, using the conditional probabilities (CPs) instead of the cumulative incidence functions as the model outcome when the feature importance is computed allows overcoming a misinterpretation of the feature importance as the outcome for one transition is conditioned by the outcome of competing outcomes. Optionally, the method comprises selecting a type of predictions among a CIFs type and a CPs type (e.g., as an option parameter for the user to choose the type of predictions. The selected type of predictions may then be used by the PFI algorithm for the computing of the quantifications.

Regarding the PDP algorithm, the method considers transition-specific partialdependence plots, uses the conditional probabilities instead of the cumulative incidence functions as the model outcome when the partial dependence plots are computed. Optionally, the method comprises selecting a type of predictions among a CIFs type and a CPs type (e.g., as an option parameter for the user to choose the type of predictions. The selected type of predictions may then be used by the PDP algorithm for the determining of the relationships.

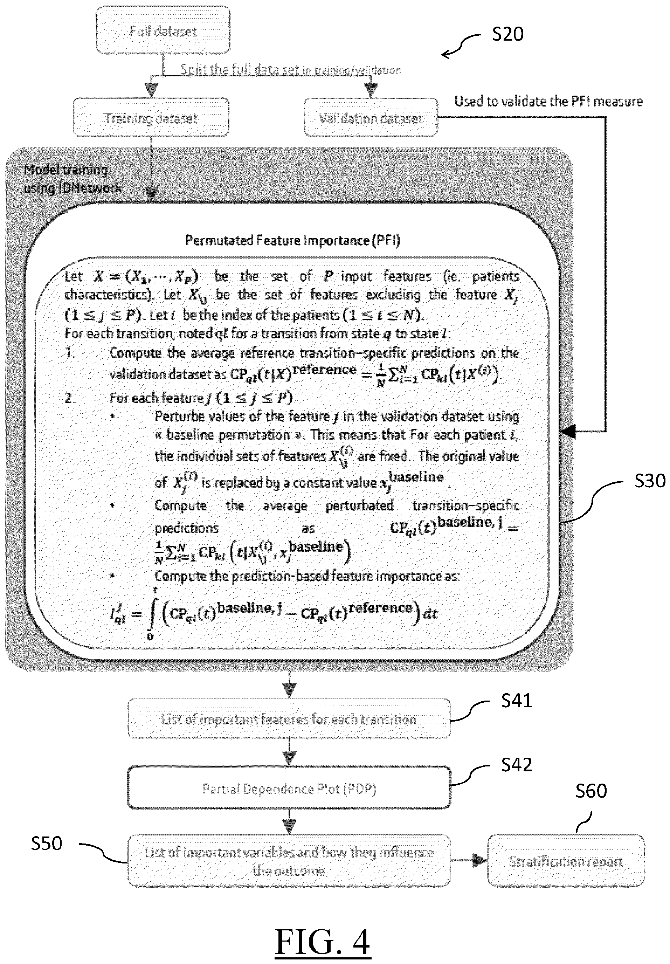

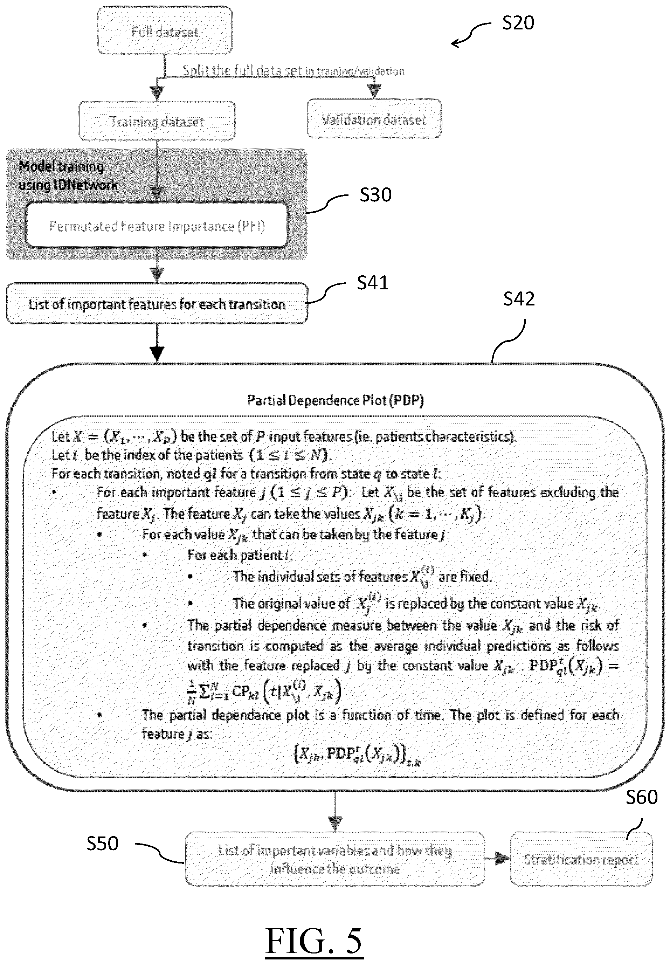

An example of implementation of the method is illustrated in FIG. 3. The method comprises the providing S10 of the deep learning neural network and the providing S20 of the dataset. The providing S20 of the dataset may comprise extracting a portion of a full dataset (the extracted portion being the provided dataset and the other portion of the full dataset being a validation dataset), as illustrated in the FIG. 3. The PFI algorithm S30 is performed during the model training. An example of the PFI algorithm S30 is illustrated in FIG. 4. After the performing of the PFI algorithm S30, the method comprises the identifying S41 of the list of characteristics for each transition and performs the PDP algorithm S42. The steps S41 and S42 are performed after the model training. An example of the PDP algorithm S40 is illustrated in FIG. 5. After the performing of the PDP algorithm S40, the method comprises the patients subgroups stratification S50 and the determination of essential characteristics for survival analysis. The method comprises creating subgroups stratification based on the transition-specific (e.g., time-based) predictions. The method uses time series clustering (e.g., time series Kmeans clustering) to create subgroups of patients based on their transition-specific risks (e.g., "risk clusters"). Then, the method comprises analyzing subgroups characteristics with a factor analysis for mixed data (FAMD). This process allows exploring patient's characteristics of each subgroup (e.g., each risk cluster) and considering potential correlations between features.

Then, the method comprises interpreting each transition of the trained model and the procedure is to classify the transitions in:

- Transitions associated with a favorable (+) clinical evolution (for instance the complete response to treatment); characteristics associated with these transitions may be used for patient selection in a clinical trial or in a care procedure (treatment, intervention, follow-up);
- Transitions associated with a negative (-) clinical evolution (for instance a relapse): characteristics associated with these transitions may be used for patient exclusion from a clinical trial or from a care procedure (treatment, intervention, follow-up).

In particular, the method may consider the positive (negative) effect of the characteristic on the transition. The method may for example be based on guidelines of interpretation for the characteristics, e.g. as formulated in the following table 1.

Table 1: Guidelines of interpretation for the characteristics

|  | Favorable Transition (+) | Unfavorable Transitions (-) |
| --- | --- | --- |
| **Positive correlation feature** | Inclusion criteria | Exclusion criteria |
| **Negative correlation feature** | Exclusion criteria | Inclusion criteria |

The method thus obtains inclusion/exclusion criteria and may comprise simulating a clinical study designed according to these inclusion/exclusion criteria. Then, the method may comprise checking that the simulated clinical study is an unbiased study. For example, the method may check that patients in control and experimental arms of the simulated trial have balanced characteristics across arms. To do so, the method may perform a verification based on propensity scores that allow to verify a good balanced of patients' characteristics between arms.

With reference to FIG. 4, an example of the determining S30 of the respective quantification is now discussed in more details.

The determining S30 of the respective quantification may comprise, for each transition $ql$ from a state q to a state $l$, computing an average reference transition-specific conditional probability based on the formula:

$$CP_{ql}(t|X)^{reference} = \frac{1}{N}\sum_{i=1}^{N} CP_{ql}\big(t|X^{(i)}\big)$$

wherein $X = (X_1, ...,X_p)$ is the set of characteristics, i is the index of each patient of the set ($1 \leq i \leq$ N), $CP_{ql}(t|X^{(i)})$ is the conditional probability for the real values of the set of characteristics $X^{(i)}$ for the patient $i$ and $CP_{ql}(t|X)^{reference}$ is the computed average reference transition-specific conditional probability.

Then, the determining S30 may comprise, for each characteristic $j$ $(1 \leq j \leq P)$, computing the following two steps. The first of the two step may consist in computing an average perturbated transition-specific conditional probability based on the formula:

$$CP_{ql}(t)^{baseline,j} = \frac{1}{N}\sum_{i=1}^{N} CP_{ql}\left(t|X_{\backslash j}^{(i)}, x_j^{baseline}\right)$$

wherein $X_{\backslash j}$ is the set of characteristics without the characteristic $X_j$ $(1 \leq j \leq P)$, $CP_{ql}\left(t|X_{\backslash j}^{(i)}, x_j^{baseline}\right)$ is a conditional probability obtained for the patient $i$ based on the baseline value $x_j^{baseline}$ for the characteristic j and the real values of the patient for each other characteristic of the set $X_{\backslash j}^{(i)}$ and $CP_{ql}(t)^{basevine,j}$ is the computed average perturbated transition-specific conditional probability.

The second of the two step may consist in computing a prediction-based feature importance based on the formula:

$$I_{ql}^j = \int_0^t \left(CP_{ql}(t)^{baseline,j} - CP_{ql}(t|X)^{reference}\right)dt$$

wherein $I_{ql}^j$ is the computed prediction-based feature importance, thereby determining the respective quantification determined for the characteristic and the transition by the method (the computed prediction-based feature importance

$I_{ql}^j$ being the respective quantification determined for the characteristic and the transition by the method).

With reference to FIG. 5, an example of the determining S42 of the respective quantification is now discussed in more details.

The determining S42, for each transition $ql$ from a state q to a state $l$, of the relationship for each characteristic of the list may comprise, for each potential value $X_{jk}$ $(k = 1, ..., K_j)$ of the characteristic $j$, computing a partial dependence measure between the potential value $X_{jk}$ and a risk of transition as an average of individual predictions. The computing of the partial dependence measure may be based on the formula:

$$PDP_{ql}^t(X_{jk}) = \frac{1}{N}\sum_{i=1}^{N} CP_{ql}\left(t|X_{\backslash j}^{(i)}, X_{jk}\right)$$

wherein $X = (X_1, ..., X_p)$ is the set of characteristics, $X_{\backslash j}$ is the set of characteristics without the characteristic $X_j$ $(1 \leq j \leq$

P), $CP_{ql}\left(t|X_{\backslash j}^{(i)}, X_{jk}\right)$ is a conditional probability obtained for the patient i based on the potential value $X_{jk}$ for the

characteristic j and the real values of the patient for each other characteristic of the set $X_{\backslash j}^{(i)}$ and $PDP_{ql}^t(X_{jk})$ is the computed partial dependence measure.

Then, the determining S42 may comprise determining a partial dependance plot based on the computed partial dependence measures. The partial dependance plot may be defined, for each characteristic $j$, as

$$\left\{ X_{jk}, PDP_{ql}^{t}\left(X_{jk}\right)\right\}_{t,k}$$

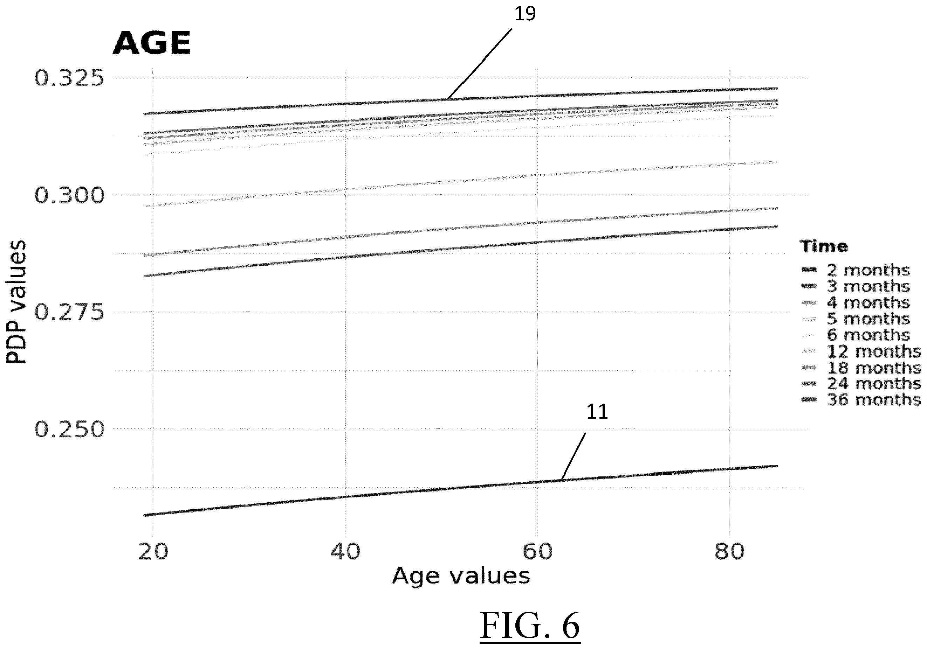

For each discrete characteristic, the potential values $X_{jk}$ ($k$ = 1, ..., $K_j$) of the characteristic $j$ considered for the computing of the relationship may be the potential values of the discrete characteristic. For each continuous characteristic, the potential values $X_{jk}$ ($k$ = 1, ..., $K_j$) of the characteristic $j$ considered for the computing of the relationship may correspond to a (e.g., exhaustive) sampling of potential values between a minimum value and a maximum value of characteristic. For example, for the characteristic "age", the method may comprise calculating a value of DPD per time between a min value of 18 years and a max value of 85 years and for several time points, thereby obtaining the different curves illustrated in FIG. 6. The curve 11 corresponds to a first time point of 2 months and the curve 19 corresponds to a last time point of 36 months. The other curves in between these curves 11 and 19 correspond to intermediate time points in between the first time point of 2 months and the last time point of 36 months.

The partial dependance plot may comprises, for each transition, a respective graphical representation. The respective graphical representation may represent, for each characteristic of the identified list of the transition, a respective curve representing the risk of transition as a function of time for the characteristic. For example, the respective graphical representation may comprise, for each characteristic of the identified list, a respective graph comprising curves each for a selected value of the characteristic. The curves of the respective graph may represent the risk of transition as a function of time for the selected values of the characteristic (each curve being associated to one of the selected value). For each discrete characteristic, the selected values may be the potential values of the discrete characteristic. For each continuous characteristic, the selected values may correspond to a (e.g., exhaustive) sampling of potential values between a minimum value and a maximum value of characteristic.

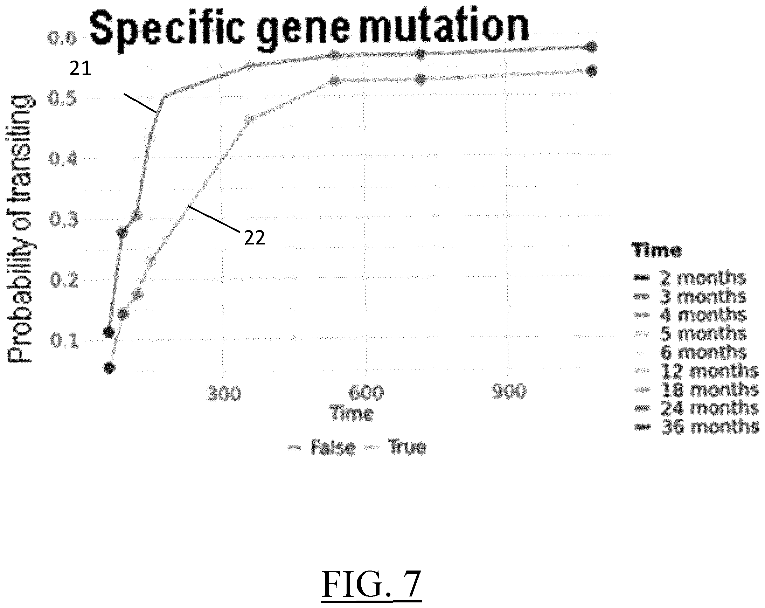

FIG. 7 shows a first example of graph for the characteristic which is the presence of a specific gene mutation. The graph comprises two curves each for a selected value of this characteristic. The first curve 21 represents the probability of transiting as a function of time when the specific gene mutation is not present (the value of the characteristic is "false"). The second curve 22 represents the probability of transiting as a function of time when the specific gene mutation is present (the value of the characteristic is "true"). The graph shows the impact of the specific gene mutation in the evolution over time of the probability of transiting.

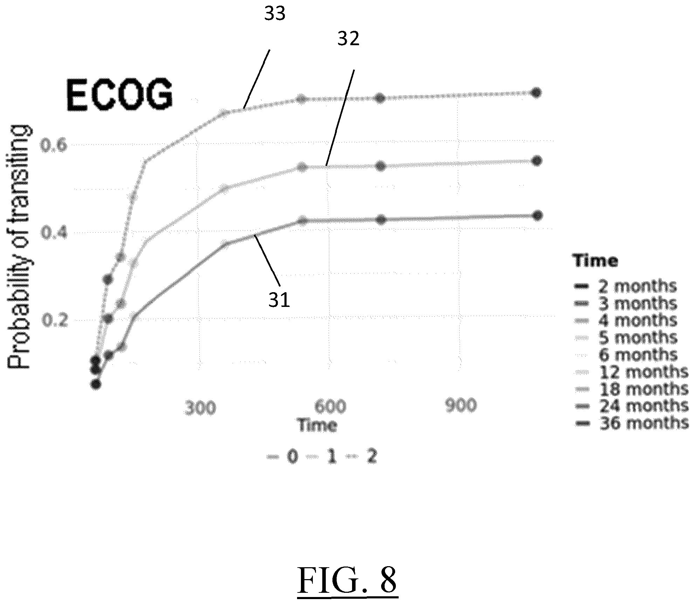

FIG. 8 shows a second example of graph for the characteristic ECOG. The graph comprises three curves each for a selected value of this characteristic. The first curve 31 represents the probability of transiting as a function of time when the characteristic ECOG has a value of 0. The second curve 32 represents the probability of transiting as a function of time when the characteristic ECOG has a value of 1. The third curve 33 represents the probability of transiting as a function of time when the characteristic ECOG has a value of 2. The graph shows the impact of the characteristic ECOG in the evolution over time of the probability of transiting.

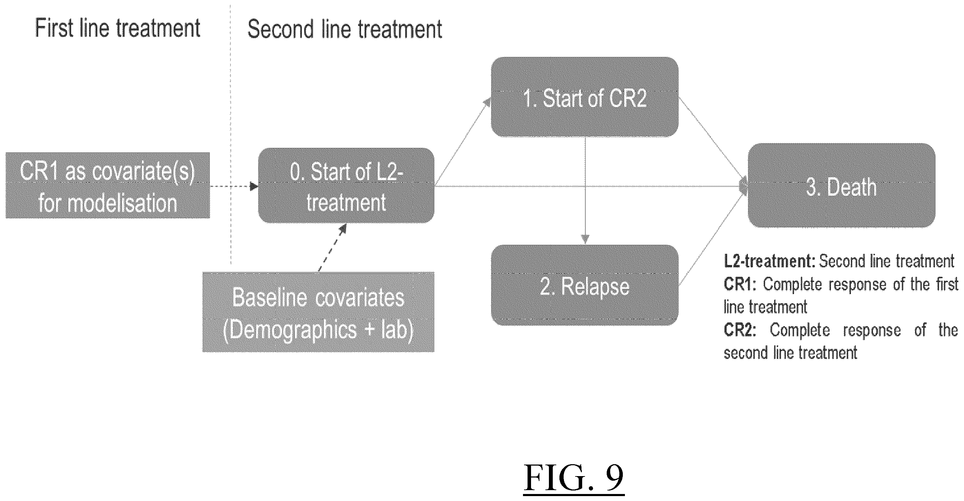

With reference to FIG. 9, a first example of implementation of the method of use in the case of the AML (Acute Myeloid Leukemia) cancer is now discussed.

The AML cancer is a blood cancer that starts in bone marrow. It exists new targeted treatment that attempts to show efficacy on relapsed patients. Showing efficacy of a new drug in late stage cancers can be hard. The disease progression of the patients may be used to understand each patient journey though the disease. Key events for patients with AML are how they respond to the treatment, i.e. "complete response to second line treatment", and if they relapse. Patients' characteristics and disease history need to be considered like for example if they respond to the previous line of treatment. Using multi-state modeling, the method of use may comprise expressing the disease progression model in terms of clinical events (see FIG. 9). The multi-state model can be trained to predict patient disease progression, and the method of use may comprise interpreting the trained model to understand clinical factors of progression. The understanding of the clinical factors of progression may lead to better clinical study decisions. In clinical development, the purpose is to show the superiority of a new treatment. When pharmaceutical companies try to put a new cancer therapy on market, they need to ensure the demonstration of a therapy efficacy against the standard of care. If they choose the wrong population or a population that is too large, they may not be successful. In this context, the method of use comprises using the pipeline of interpretability algorithms of the method on this specific multi-state model. That leads to understand which patient characteristics impact the disease progression. These patient characteristics will allow to specify a subpopulation of patients that benefit the treatment. Targeting this population increases the chance of success for new drug approval that will be efficient in a targeted population of patients.

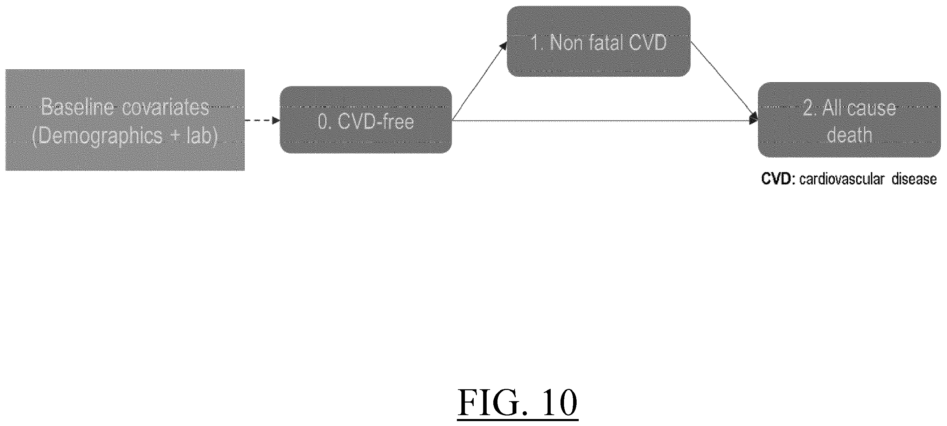

With reference to FIG. 10, a second example of implementation of the method of use in the case of hypertension ([23]) is now discussed.

For common diseases like hypertension, it can be difficult to show improvement in new protocols of care or new indications for a large population of patients. Patients with hypertension are heterogeneous and causes of CVD (cardiovascular disease) needs to be understood (see FIG. 10). The method of use, by using multi-state modeling to model the patient journey through the disease combined with the interpretability pipeline, helps understanding patient characteristics that lead to CVD. This can help for prevention of CVD events.

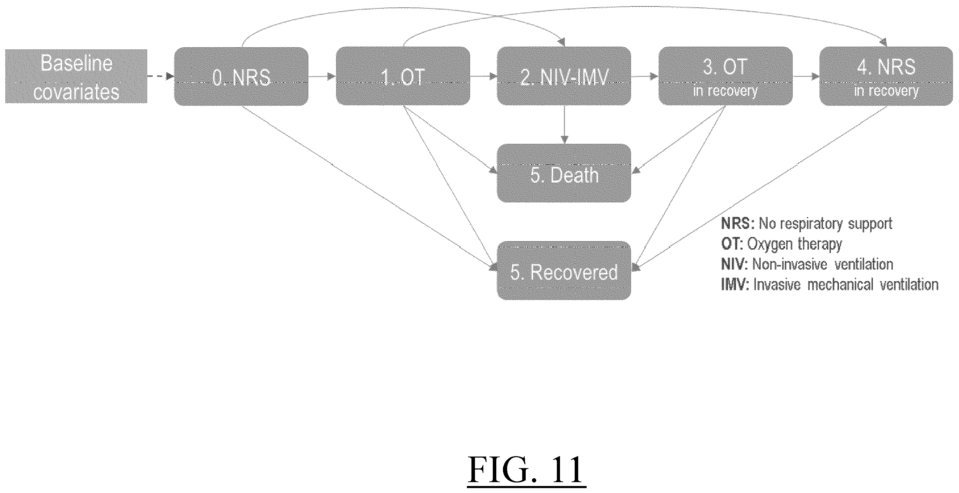

With reference to FIG. 11, a third example of implementation of the method of use in the case of infectious disease COVID-19 ([24]) is now discussed.

For more complex diseases like COVID-19, diseased patients can experience many events that are crucial to consider for modeling disease progression (see FIG. 11). Classical survival analysis does not allow to understand the complexity of disease progression. The method of use, by using multi-state modeling and adapted interpretability algorithms, allows to understand the patient journey. This helps identifying patient characteristics that increase the risk of disease progression and identifying patients who are likely to need a specific respiratory support as invasive or non-invasive ventilation for example

The following table 2 provides other examples of disease that may be modeled by the multi-state model, and that the method may interpret for identifying patient characteristics that influence the disease progression.

Table 2: Other examples of disease

| Disease | Reference |
|---|---|
| Hypertension | Ramezankhani, A., Blaha, M.J., Mirbolouk, M.h. et al. Multi-state analysis of hypertension and mortality: application of semi-Markov model in a longitudinal cohort study. BMC Cardiovasc Disord 20, 321 (2020). |
| Lupus | Smith, E. M. D., Eleuteri, A., Goilav, B., Lewandowski, L., Phuti, A., Rubinstein, T., ... & Beresford, M. W. (2019). A Markov Multi-State model of lupus nephritis urine biomarker panel dynamics in children: Predicting changes in disease activity. Clinical Immunology, 198, 71-78. |
| Infectious Disease COVID-19 | Milic, J., Banchelli, F., Meschiari, M., Franceschini, E., Ciusa, G., Gozzi, L., ... & D'Amico, R. (2021). The impact of tocilizumab on respiratory support states transition and clinical outcomes in |
| | COVID-19 patients. A Markov model multi-state study. PloS one, 16(8), e0251378. |
| Gastric Cancer | Zare, A., Mahmoodi, M., Mohammad, K., Zeraati, H., Hosseini, M., & Naieni, K. H. (2013). Comparison between parametric and semi-parametric cox models in modeling transition rates of a multi-state model: application in patients with gastric cancer undergoing surgery at the Iran cancer institute. Asian Pacific Journal of Cancer Prevention, 14(11), 6751-6755. |
| Infectious disease | Van Kleef, E., Green, N., Goldenberg, S. D., Robotham, J. V., Cookson, B., Jit, M., ... & Deeny, S. R. (2014). Excess length of stay and mortality due to Clostridium difficile infection: a multi-state modelling approach. Journal of Hospital Infection, 88(4), 213-217. |
| Heart Failure | Gasperoni, F., Ieva, F., Barbati, G., Scagnetto, A., Iorio, A., Sinagra, G., & Di Lenarda, A. (2017). Multi-state modelling of heart failure care path: a population-based investigation from Italy. PloS one, 12(6), e0179176. |
| Addiction | Mayet, A., Legleye, S., Falissard, B., & Chau, N. (2012). Cannabis use stages as predictors of subsequent initiation with other illicit drugs among French adolescents: use of a multi-state model. Addictive behaviors, 37(2), 160-166. |
| HIV | Gentleman, R. C., Lawless, J. F., Lindsey, J. C., & Yan, P. (1994). Multi-state Markov models for analysing incomplete disease history data with illustrations for HIV disease. Statistics in medicine, 13(8), 805-821. |
| Tuberculosis | Graham, J., Smith, G. C., Delahay, R. J., Bailey, T., McDonald, R. A., & Hodgson, D. (2013). Multi-state modelling reveals sexdependent transmission, progression and severity of tuberculosis in wild badgers. Epidemiology & Infection, 141(7), 1429-1436. |

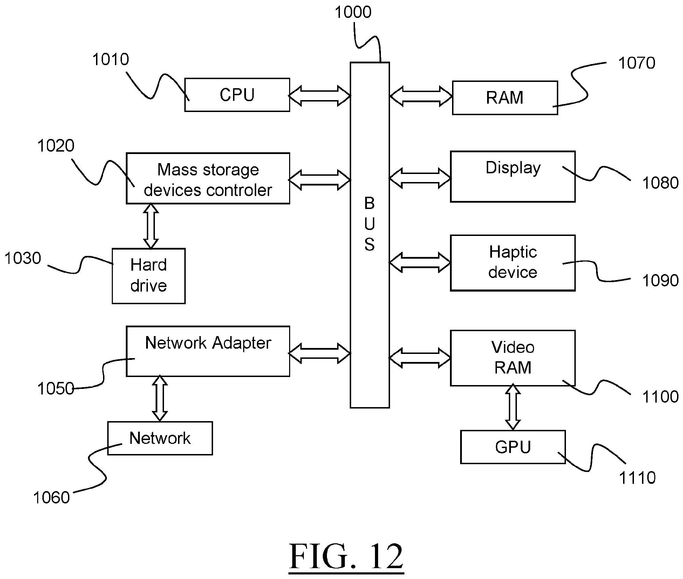

FIG. 12 shows an example of the system, wherein the system is a client computer system, e.g. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

**Claims**

1. A computer-implemented method for determining characteristics of a patient that influence a progression of a disease of the patient, the method comprising:

   • providing (S10) a deep learning neural network modeling a multi-state model of the disease progression comprising states and transitions between the states, the multi-state model outputting transition-specific probabilities during a follow-up period;
   • providing (S20) a dataset of multi-state time-to-event data of a set of patients, the dataset comprising, for each patient of the set, a real value of each characteristic of a set of characteristics;
   • while training the neural network with the provided dataset, for each transition of the multi-state model:

      ○ for each characteristic, determining (S30) a respective quantification of an impact of the characteristic on the results of the neural network, the determined respective quantification being a sum, for each patient, of a difference between a conditional probability obtained for a baseline value of the characteristic and a conditional probability obtained for the real value of the characteristic for the patient; and

   • for each transition:

      ○ identifying (S41) a list of characteristics of the set of characteristics, each characteristic of the list having a significant respective quantification for the transition;
      ○ for each given characteristic of the identified list, determining (S42) a relationship between the given characteristic and probabilities of transition based on computed predictions of conditional probabilities for each patient of the set of patients, the relationship being determined by fixing each characteristic of the patient to each value of the characteristic; and

   • providing (S50) the identified lists and the determined relationships that influence the progression of the disease of the patient.

2. The method of claim 1, wherein the determining (S30) of the respective quantification comprises, for each transition $ql$ from a state $q$ to a state $l$:

   • computing an average reference transition-specific conditional probability based on the formula:

$$CP_{ql}(t|X)^{reference} = \frac{1}{N}\sum_{i=1}^{N} CP_{ql}\left(t|X^{(i)}\right)$$

wherein $X = (X_1, ..., X_p)$ is the set of characteristics, $i$ is the index of each patient of the set $(1 \le i \le N)$, $CP_{ql}(t|X^{(i)})$ is the conditional probability for the real values of the set of characteristics $X^{(i)}$ for the patient $i$ and $CP_{ql}(t|X)^{reference}$ is the computed average reference transition-specific conditional probability; and

• For each characteristic $j$ $(1 \le j \le P)$:

○ computing an average perturbated transition-specific conditional probability based on the formula:

$$CP_{ql}(t)^{baseline,j} = \frac{1}{N}\sum_{i=1}^{N} CP_{ql}\left(t|X_{\backslash j}^{(i)}, x_j^{baseline}\right)$$

wherein $X_{\backslash j}$ is the set of characteristics without the characteristic $X_j$ $(1 \le j \le P)$, $CP_{ql}\left(t|X_{\backslash j}^{(i)}, x_j^{baseline}\right)$ is a conditional probability obtained for the patient $i$ based on the baseline value $x_j^{baseline}$ for the characteristic $j$ and the real values of the patient for each other characteristic of the set $X_{\backslash j}^{(i)}$ and $CP_{ql}(t)^{baseline,j}$ is the computed average perturbated transition-specific conditional probability; and

○ computing a prediction-based feature importance based on the formula:

$$I_{ql}^{j} = \int_{0}^{t} \left(CP_{ql}(t)^{baseline,j} - CP_{ql}(t|X)^{reference}\right)dt$$

wherein $I_{ql}^{j}$ is the computed prediction-based feature importance, thereby determining the respective quantification for the characteristic and the transition.

3. The method of claim 1 or 2, wherein the baseline value of the characteristic is the same for each of the patients.

4. The method of any one of claims 1 to 3, wherein the set of characteristics comprise one or more discrete characteristics and/or one or more continuous characteristics, the real value of each patient being, for each of the one or more discrete characteristics, one of a respective predetermined set of potential values, the baseline value being:

• for each discrete characteristic, one of the potential values of the respective predetermined set of the discrete characteristic, and
• for each continuous characteristic, determined according to a scaling of real values of the patients for the characteristic.

5. The method of claim 4, wherein the determining (S42), for each transition $ql$ from the state $q$ to the state $l$, of the relationship for each characteristic of the list comprising:

• for each potential value $X_{jk}$ $(k = 1, ..., K_j)$ of the characteristic $j$:

○ computing a partial dependence measure between the potential value $X_{jk}$ and a risk of transition as an average of individual predictions based on the formula:

$$PDP_{ql}^t(X_{jk}) = \frac{1}{N}\sum_{i=1}^{N} CP_{ql}\left(t|X_{\setminus j}^{(i)}, X_{jk}\right)$$

wherein $X = (X_1, ..., X_p)$ is the set of characteristics, $X_{\setminus j}$ is the set of characteristics without the characteristic $X_j$ $(1 \leq j \leq P)$, $CP_{ql}\left(t|X_{\setminus j}^{(i)}, X_{jk}\right)$ is a conditional probability obtained for the patient $i$ based on the potential value $X_{jk}$ for the characteristic $j$ and the real values of the patient for each other characteristic of the set $X_{\setminus j}^{(i)}$ and $PDP_{ql}^t(X_{jk})$ is the computed partial dependence measure; and

• determining a partial dependance plot based on the computed partial dependence measures, the partial dependance plot being defined, for each characteristic $j$, as $\left\{X_{jk}, PDP_{ql}^t(X_{jk})\right\}_{t,k}$ .

6. The method of any one of claim 5, wherein the partial dependance plot comprises, for each transition, a respective graphical representation, the respective graphical representation representing, for each characteristic of the identified list of the transition, a respective curve representing the risk of transition as a function of time for the characteristic.

7. The method of any one of claims 1 to 6, wherein the multi-state model is an illness-death model.

8. The method of any one of claims 1 to 7, further comprising:

• while training the neural network with the provided dataset, for each transition of the multi-state model:

∘ for each characteristic, determining a respective performance quantification of an impact of the characteristic on performances of the neural network;

• for each transition:

∘ identifying another list of characteristics of the set of characteristics, each characteristic of the another list having a significant respective performance quantification for the transition;

• selecting, by user interaction, an analysis type among a result type and a performance type, the result type being associated to the list and the performance type being associated to the another list; and
• providing to the user the lists associated to the analysis type selected by the user.

9. The method of any one of claims 1 to 8, wherein a respective quantification of a characteristic is significant when the respective quantification is positive for at least 50% of the patients, preferably 95% of the patients.

10. A method of use of characteristics of the patient that influence the progression of the disease of the patient determined by the method of any one of claims 1 to 9, the method comprising performing subgroups stratification (S60) of patients based on the provided lists and relationships that influence the progression of the disease of the patient.

11. The method of claim 10, further comprising:

• classifying each transition in a first group of transitions or a second group of transitions, the transitions of the first group being associated with a favorable clinical evolution and the transitions of the second group being associated with a negative clinical evolution; and
• determining inclusion and exclusion criteria for a survival analysis based on the classified transitions.

12. The method of claim 10, further comprising determining a follow-up period for one or more patients, the determining of the follow-up period comprising, for each patient:

• determining the subgroup of the patient; and

• determining a follow-up period for the patient according to the determined subgroup of the patient.

13. A computer program comprising instructions which, when the program is executed by a computer, cause the computer to carry out the method of any one of claims 1 to 9 and/or the method of use of any one of claims 10 to 12.

14. A computer readable storage medium having recorded thereon the computer program of claim 13.

15. A system comprising a processor coupled to a memory, the memory having recorded thereon the computer program of claim 13.

Providing a deep learning neural network modeling a multi-state model of the disease progression — S10

Providing a dataset of multi-state time-to-event data of a set of patients — S20

While training the neural network with the provided dataset, for each transition of the multi-state model, determining a respective quantification of an impact of each characteristic — S30

For each transition: — S40

Identifying a list of characteristics — S41

For each given characteristic of the identified list, determining a relationship between the given characteristic and probabilities of transition — S42

Providing the identified respective lists and the determined relationships that influence the progression of the disease of the patient — S50

FIG. 1

FUNDAMENTALS OF DESIGN FOR MAJOR RANDOMIZED CONTROLLED TRIALS

**Choice of patients and centers**
- Set precise eligibility criteria, neither too specific nor too broad
- Large-scale, multicenter trials are preferable
- Choose appropriate geographic representation

**Choice of treatments**
- Specify precise treatment regimens
- Specify placebo/sham control group or an active comparator
- Sometimes a 3-arm trial is appropriate

**Choice of outcomes**
- Define the primary efficacy endpoint
- Take care in selecting components of composite primary endpoint
- List secondary endpoints
- Incorporate pre-defined safety concerns into overall outcome priorities

**Randomization**
- Allocation concealment is crucial
- Choose a statistical method for randomization
- Stratifying helps to ensure groups are balanced but not key in large trials
- Unequal randomization in favor of new treatment sometimes useful

**Use of blinding**
- Make trial double-blind if practical
- If not practical, blinded evaluation is required
- Blinding especially important for "softer" endpoints

**Choice of trial size**
- Use power calculations to determine required trial size
- Choose a realistic anticipated effect size
- Compromise is needed in making the target size achievable for a real world study
- Useful to determine how many primary events are needed

Pocock, S.J. et al. J Am Coll Cardiol. 2015; 66(24):2757-66.

FIG. 2

**FIG. 3**

Full dataset

Split the full data set in training/validation — S20

Training dataset

Validation dataset

Used to validate the PFI measure

**Model training using IDNetwork**

**Permutated Feature Importance (PFI)**

Let $X = (X_1, \cdots, X_P)$ be the set of $P$ input features (ie. patients characteristics). Let $X_{\backslash j}$ be the set of features excluding the feature $X_j$ $(1 \leq j \leq P)$. Let $i$ be the index of the patients $(1 \leq i \leq N)$.

For each transition, noted $ql$ for a transition from state $q$ to state $l$:

1. Compute the average reference transition–specific predictions on the validation dataset as $CP_{ql}(t|X)^{\text{reference}} = \frac{1}{N} \sum_{i=1}^{N} CP_{kl}(t|X^{(i)})$.

2. For each feature $j$ $(1 \leq j \leq P)$
   - Perturbe values of the feature $j$ in the validation dataset using « baseline permutation ». This means that For each patient $i$, the individual sets of features $X_{\backslash j}^{(i)}$ are fixed. The original value of $X_j^{(i)}$ is replaced by a constant value $x_j^{\text{baseline}}$.
   - Compute the average perturbated transition–specific predictions as $CP_{ql}(t)^{\text{baseline, j}} = \frac{1}{N} \sum_{i=1}^{N} CP_{kl}\left(t|X_{\backslash j}^{(i)}, x_j^{\text{baseline}}\right)$
   - Compute the prediction-based feature importance as:
   
   $$I_{ql}^j = \int_0^t \left(CP_{ql}(t)^{\text{baseline, j}} - CP_{ql}(t)^{\text{reference}}\right) dt$$

S30

List of important features for each transition — S41

Partial Dependence Plot (PDP) — S42

S50 — List of important variables and how they influence the outcome

S60

Stratification report

# FIG. 4

Full dataset

S20

Split the full data set in training/validation

Training dataset

Validation dataset

**Model training using IDNetwork**

Permutated Feature Importance (PFI)

S30

List of important features for each transition

S41

S42

Partial Dependence Plot (PDP)

Let $X = (X_1, \cdots, X_p)$ be the set of $P$ input features (ie. patients characteristics).
Let $i$ be the index of the patients ($1 \leq i \leq N$).
For each transition, noted $ql$ for a transition from state $q$ to state $l$:

- For each important feature $j$ ($1 \leq j \leq P$): Let $X_{\backslash j}$ be the set of features excluding the feature $X_j$. The feature $X_j$ can take the values $X_{jk}$ ($k = 1, \cdots, K_j$).
  - For each value $X_{jk}$ that can be taken by the feature $j$:
    - For each patient $i$,
      - The individual sets of features $X_{\backslash j}^{(i)}$ are fixed.
      - The original value of $X_j^{(i)}$ is replaced by the constant value $X_{jk}$.
    - The partial dependence measure between the value $X_{jk}$ and the risk of transition is computed as the average individual predictions as follows with the feature replaced $j$ by the constant value $X_{jk}$ : $\mathrm{PDP}_{ql}^t(X_{jk}) = \frac{1}{N}\sum_{i=1}^{N} \mathrm{CP}_{kl}\left(t | X_{\backslash j}^{(i)}, X_{jk}\right)$
  - The partial dependance plot is a function of time. The plot is defined for each feature $j$ as: $$\{X_{jk}, \mathrm{PDP}_{ql}^t(X_{jk})\}_{t,k}.$$

S50

List of important variables and how they influence the outcome

S60

Stratification report

## FIG. 5

FIG. 6

FIG. 7

EP 4 386 767 A1

FIG. 8

First line treatment | Second line treatment

CR1 as covariate(s) for modelisation

0. Start of L2-treatment

1. Start of CR2

2. Relapse

3. Death

Baseline covariates (Demographics + lab)

**L2-treatment:** Second line treatment
**CR1:** Complete response of the first line treatment
**CR2:** Complete response of the second line treatment

FIG. 9

FIG. 10

CVD: cardiovascular disease

Baseline covariates

0. NRS

1. OT

2. NIV-IMV

3. OT in recovery

4. NRS in recovery

5. Death

5. Recovered

NRS: No respiratory support
OT: Oxygen therapy
NIV: Non-invasive ventilation
IMV: Invasive mechanical ventilation

FIG. 11

FIG. 12

Europäisches
Patentamt

European
Patent Office

Office européen
des brevets

# EUROPEAN SEARCH REPORT

Application Number

EP 22 30 6927

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/366577 A1 (KOLLER DAPHNE [US] ET AL) 25 November 2021 (2021-11-25) * paragraphs [0006], [0025], [0005], [0128], [0271], [0322], [0217], [0182] * | 1-15 | INV. G16H10/20 G16H50/20 G16H50/70 |
| A | Anonymous: "Highly Specialised Technology Evaluation Asfotase alfa for treating paediatric-onset hypophosphatasia [ID 758]", Evaluation Report (Committee Papers), 21 October 2015 (2015-10-21), pages 1-752, XP055613436, GB Retrieved from the Internet: URL:https://www.nice.org.uk/guidance/hst6/documents/committee-papers-8 [retrieved on 2023-04-11] * page 1 * * paragraph [5.3.2] - paragraph [5.3.3ff] * | 1-15 | |
| A | CERNOCH PATRICK S ET AL: "Lower Risk of B1-to-pB3-Stage Migration in Crohn's Disease Upon Immunosuppressive and Anti-TNF Treatment in the Swiss IBD Cohort Study", DIGESTIVE DISEASES AND SCIENCES, SPRINGER NEW YORK LLC, US, vol. 65, no. 9, 3 December 2019 (2019-12-03), pages 2654-2663, XP037216455, ISSN: 0163-2116, DOI: 10.1007/S10620-019-05978-9 [retrieved on 2019-12-03] * pages 2656, 26 * | 1-15 | TECHNICAL FIELDS SEARCHED (IPC) G16H |

-/--

The present search report has been drawn up for all claims

| Place of search | Date of completion of the search | Examiner |
|---|---|---|
| Munich | 30 May 2023 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

**EP 22 30 6927**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHIH S T F ET AL: "Cost-effectiveness Analysis of a Two-stage Screening Intervention for Hepatocellular Carcinoma in Taiwan", JOURNAL OF THE FORMOSAN MEDICAL ASSOCIATION, EXCERPTA MEDICA ASIA, HONG KONG, HK, vol. 109, no. 1, 1 January 2010 (2010-01-01), pages 39-55, XP026914010, ISSN: 0929-6646, DOI: 10.1016/S0929-6646(10)60020-4 [retrieved on 2010-01-01] * the whole document * | 1-15 | |
| A | CHRISTOPHER H. JACKSON ET AL: "Multistate Markov models for disease progression with classification error", STATISTICIAN, vol. 52, no. 2, 1 July 2003 (2003-07-01), pages 193-209, XP055276568, GB ISSN: 0039-0526, DOI: 10.1111/1467-9884.00351 * the whole document * | 1-15 | **TECHNICAL FIELDS SEARCHED (IPC)** |

The present search report has been drawn up for all claims

| Place of search | Date of completion of the search | Examiner |
|---|---|---|
| Munich | 30 May 2023 | Laub, Christoph |

EPO FORM 1503 03.82 (P04C01)

## ANNEX TO THE EUROPEAN SEARCH REPORT
## ON EUROPEAN PATENT APPLICATION NO.

EP 22 30 6927

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2023

| Patent document cited in search report | | Publication date | Patent family member(s) | | Publication date |
|---|---|---|---|---|---|
| US 2021366577 | A1 | 25-11-2021 | AU 2021275995 | A1 | 19-01-2023 |
| | | | CA 3178602 | A1 | 25-11-2021 |
| | | | CN 115698335 | A | 03-02-2023 |
| | | | EP 4153782 | A1 | 29-03-2023 |
| | | | KR 20230015408 | A | 31-01-2023 |
| | | | US 2021366577 | A1 | 25-11-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 4057297 A1

**Non-patent literature cited in the description**

- **PER K ANDERSEN ; ORNULF BORGAN ; RICHARD D GILL ; NIELS KEIDING.** Statistical models based on counting processes. Springer Science & Business Media, 2012
- **DAVID R COX.** Regression models and lifetables. *Journal of the Royal Statistical Society: Series B (Methodological),* 1972, vol. 34 (2), 187-202
- **STUART J. POCOCK, PHD ; TIM C. CLAYTON, MSC ; GREGG W. STONE, MD.** *Design of Major Randomized Trials: Part 3 of a 4-Part Series on Statistics for Clinical Trials,* 2015
- **GARSON, G.D.** Interpreting neural network connection weights. *Artificial Intelligence Expert,* 1991, vol. 6 (4), 46-51
- **CHRISTOPH MOLNAR.** *Interpretable machine learning,* 2020
- Random Forests. **BREIMAN, LEO.** Machine Learning. Springer, 2001, vol. 45, 5-32
- Distribution-Free Predictive Inference for Regression. **LEI, JING ; MAX G'SELL ; ALESSANDRO RINALDO ; RYAN J TIBSHIRANI ; LARRY WASSERMAN.** Journal of the American Statistical Association. Taylor & Francis, 2018, vol. 113, 1094-1111
- **FRIEDMAN, JEROME H.** Greedy function approximation: A gradient boosting machine. *Annals of statistics,* 2001, 1189-1232
- **APLEY, DANIEL W. ; JINGYU ZHU.** Visualizing the effects of predictor variables in black box supervised learning models. *Journal of the Royal Statistical Society: Series B (Statistical Methodology),* 2020, vol. 82.4, 1059-1086
- **GOLDSTEIN, ALEX ; ADAM KAPELNER ; JUSTIN BLEICH ; EMIL PITKIN.** Peeking inside the black box: Visualizing statistical learning with plots of individual conditional expectation. *journal of Computational and Graphical Statistics,* 2015, vol. 24 (1), 44-65
- **TULIO RIBEIRO, M. ; SINGH, S. ; GUESTRIN, C.** Why Should I Trust You?. *Explaining the Predictions of Any Classifier,* 2016

- **LUNDBERG, SCOTT M. ; SU-IN LEE.** A unified approach to interpreting model predictions. *Advances in Neural Information Processing Systems,* 2017
- **C MOLNAR ; S GRUBER ; P KOPPER.** *Limitations of interpretable machine learning methods,* 2020
- **JOHN EHRLINGER.** ggrandomforests : Exploring random forest survival. *arXiv preprint arXiv :1612.08974,* 2016
- **MAXIM S KOVALEV ; LEV V UTKIN ; ERNEST M KASIMOV.** Survlime : A method for explaining machine learning survival models. *Knowledge-Based Systems,* 2020, vol. 203, 106164
- **RICHARD LI ; ASHWIN SHINDE ; AN LIU ; SCOTT GLASER ; YUNG LYOU ; BERTRAM YUH ; JEFFREY WONG ; ARYA AMINI.** Machine learning-based interpretation and visualization of nonlinear interactions in prostate cancer survival. *JCO Clinical Cancer Informatics,* 2020, vol. 4, 637-646
- **COTTIN, A. ; PECUCHET, N. ; ZULIAN, M. ; GUILLOUX, A. ; KATSAHIAN, S.** IDNetwork: A deep illness-death network based on multi-state event history process for disease prognostication. *Statistics in Medicine,* 2022, vol. 41 (9), 1573-1598
- **ISHWARAN H ; KOGALUR UB.** Random survival forests for r. *R news,* 2007, vol. 7 (2), 25-31
- **ISHWARAN H ; GERDS TA ; KOGALUR UB et al.** Random survival forests for competing risks. *Biostatistics,* 2014, vol. 15 (4), 757-773
- **JACQMIN-GADDA H ; BLANCHE P ; CHARY E ; TOURAINE C ; DARTIGUES J-F.** Receiver operating characteristic curve estimation for time to event with semicompeting risks and interval censoring. *Stat Methods Med Res.,* 2016, vol. 25 (6), 2750-2766
- **SPITONI C ; LAMMENS V ; PUTTER H.** Prediction errors for state occupation and transition probabilities in multi-state models. *Biom J.,* 2018, vol. 60 (1), 34-48
- **PEPE MS ; MORI M.** Kaplan-meier, marginal or conditional probability curves in summarizing competing risks failure time data?. *Statistics in medicine,* 1993, vol. 12 (8), 737-751

- **RAMEZANKHANI, A. ; BLAHA, M.J. ; MIRBO-LOUK, M.H. et al.** Multi-state analysis of hypertension and mortality: application of semi-Markov model in a longitudinal cohort study. *BMC Cardiovasc Disord,* 2020, vol. 20, 321, https://doi.org/10.1186/s12872-020-01599-7
- **MILIC J ; BANCHELLI F ; MESCHIARI M ; FRANCESCHINI E ; CIUSA G et al.** The impact of tocilizumab on respiratory support states transition and clinical outcomes in COVID-19 patients. A Markov model multi-state study. *PLOS ONE,* 2021, vol. 16 (8), e0251378, https://doi.org/10.1371/journal.pone.0251378
- **RAMEZANKHANI, A. ; BLAHA, M.J. ; MIRBO-LOUK, M.H. et al.** Multi-state analysis of hypertension and mortality: application of semi-Markov model in a longitudinal cohort study. *BMC Cardiovasc Disord,* 2020, vol. 20, 321
- **SMITH, E. M. D. ; ELEUTERI, A. ; GOILAV, B. ; LEWANDOWSKI, L. ; PHUTI, A. ; RUBINSTEIN, T. ; BERESFORD, M. W.** A Markov Multi-State model of lupus nephritis urine biomarker panel dynamics in children: Predicting changes in disease activity. *Clinical Immunology,* 2019, vol. 198, 71-78
- **MILIC, J., BANCHELLI, F., MESCHIARI, M., FRANCESCHINI, E., CIUSA, G., GOZZI, L., D'AMICO, R.** The impact of tocilizumab on respiratory support states transition and clinical outcomes in COVID-19 patients. A Markov model multi-state study. *PloS one,* 2021, vol. 16 (8), e0251378
- **ZARE, A. ; MAHMOODI, M. ; MOHAMMAD, K. ; ZERAATI, H. ; HOSSEINI, M. ; NAIENI, K. H.** Comparison between parametric and semi-parametric cox models in modeling transition rates of a multi-state model: application in patients with gastric cancer undergoing surgery at the Iran cancer institute. *Asian Pacific Journal of Cancer Prevention,* 2013, vol. 14 (11), 6751-6755
- **VAN KLEEF, E. ; GREEN, N. ; GOLDENBERG, S. D. ; ROBOTHAM, J. V. ; COOKSON, B. ; JIT, M. ; DEENY, S. R.** Excess length of stay and mortality due to Clostridium difficile infection: a multi-state modelling approach. *Journal of Hospital Infection,* 2014, vol. 88 (4), 213-217
- **GASPERONI, F. ; LEVA, F. ; BARBATI, G. ; SCAGNETTO, A. ; LORIO, A. ; SINAGRA, G. ; DI LENARDA, A.** Multi-state modelling of heart failure care path: a population-based investigation from Italy. *PloS one,* 2017, vol. 12 (6), e0179176
- **MAYET, A. ; LEGLEYE, S. ; FALISSARD, B. ; CHAU, N.** Cannabis use stages as predictors of subsequent initiation with other illicit drugs among French adolescents: use of a multi-state model. *Addictive behaviors,* 2012, vol. 37 (2), 160-166
- **GENTLEMAN, R. C. ; LAWLESS, J. F. ; LINDSEY, J. C. ; YAN, P.** Multi-state Markov models for analysing incomplete disease history data with illustrations for HIV disease. *Statistics in medicine,* 1994, vol. 13 (8), 805-821
- **GRAHAM, J. ; SMITH, G. C. ; DELAHAY, R. J. ; BAILEY, T. ; MCDONALD, R. A. ; HODGSON, D.** Multi-state modelling reveals sexdependent transmission, progression and severity of tuberculosis in wild badgers. *Epidemiology & Infection,* 2013, vol. 141 (7), 1429-1436